# EUROPEAN PATENT SPECIFICATION

(11) **EP 1 335 907 B1**
(45) Date of publication and mention of the grant of the patent: **09.06.2010**
(21) Application number: 01996532.6
(22) Date of filing: 20.11.2001
(51) Int. Cl.: C07D 241/18, C07D 241/20, C07D 405/12, C07D 213/82, C07D 213/60, C07D 213/64, C07D 213/74

(54) **PIPERAZINYLPYRAZINE COMPOUNDS AS AGONIST OR ANTAGONIST OF SEROTONIN 5HT-2 RECEPTOR**
PIPERAZINYLPYRAZINVERBINDUNGEN ALS AGONISTEN ODER ANTAGONISTEN AM SEROTONIN-5HT-2-REZEPTOR
COMPOSES DE PIPERAZINYLPYRAZINE UTILISES COMME AGONISTES OU ANTAGONISTES DU RECEPTEUR DE LA SEROTONINE 5HT-2

(30) Priority: 20.11.2000 SE 0004245; 28.11.2000 US 253509 P
(43) Date of publication of application: 20.08.2003
(73) Proprietor: Biovitrum AB (publ), 112 76 Stockholm (SE)
(72) Inventor: NILSSON, Björn, S-112 49 Stockholm (SE)
(74) Representative: Höglund, Lars
(86) International application number: PCT/SE2001/002569
(87) International publication number: WO 2002/040456

(56) References cited:
- EP-A1- 0 345 808
- EP-A1- 0 462 638
- EP-A1- 1 023 898
- WO-A1-00/17163
- WO-A1-00/50401
- WO-A1-94/11363
- WO-A1-98/42712
- WO-A1-99/11619
- MALGORZATA DUKAT ET AL.: 'Structure-activity relationships for the binding of arylpiperazineses and arylbiguanides at 5-HT3 serotonin receptors' J. MED. CHEM. vol. 39, 1996, pages 4017 - 4026, XP002908104
- GREGORY M. SHUTSKE ET AL.: 'Synthesis of some piperazinylpyrazolo(3,4-b)pyridines as selective serotonin re-uptake inhibitors' J. HETEROCYCLIC CHEM. vol. 34, 1997, pages 789 - 795, XP002908108
- R.J. RODGERS ET AL.: 'Differential effects of novel ligands for 5-HT receptor subtypes on nonopioid defensive analgesia in male mice' NEUROSCIENCE & BIOBEHUVIORAL REVIEWS vol. 15, 1991, pages 489 - 495, XP002908107
- HERBERT Y. MELTZER: 'Clinical studies on the mechanism of action of clozapine: the dopamine-serotonin hypothesis of schizophrenia' PSYCHOPHARMACOLOGY vol. 99, 1989, pages 18 - 27, XP002908106
- HONG SHICK LEE ET AL.: 'Effect of the serotonin agonist, MK-212, on body temperature in schizophrenia' BIOL. PSYCHIATRY vol. 31, 1992, pages 460 - 470, XP002908105
- WILLIAM C. LUMMA, JR. ET AL.: 'Piperazinylpyrazines with central serotonin mimetic activity' JOURNAL OF MEDICINAL CHEMISTRY vol. 21, no. 6, 1978, pages 536 - 542, XP002933691
- PATENT ABSTRACTS OF JAPAN & JP 07 300 474 A (ASAHI CHEM IND CO LTD) 14 November 1995
- BRYAN L. ROTH ET AL.: 'The multiplicity of serotonin receptors: uselessly diverse molecules or an embarrassment of riches' NEUROSCIENTIST vol. 6, no. 4, 2000, pages 252 - 262, XP002948187

## Description

### Field of the Invention

The present invention relates to novel compounds, to pharmaceutical compositions comprising the compounds, to processes for their preparation, as well as to the use of the compounds for the preparation of a medicament which particularly acts on the central nervous system.

### Background of the Invention

Many diseases of the central nervous system are influenced by the adrenergic, the dopaminergic, and the serotonergic neurotransmitter systems. For example, serotonin has been implicated in a number of diseases and conditions which originate in the central nervous system. A number of pharmacological and genetic experiments involving receptors for serotonin strongly implicate the 5-HT_{2c} receptor subtype in the regulation of food intake (Obes. Res. 1995, 3, Suppl. 4, 449S-462S). The 5-HT_{2c} receptor subtype is transcribed and expressed in hypothalamic structures associated with appetite regulation. It has been demonstrated that the 5-HT_{2c} receptor agonist *m*-chlorophenylpiperazine (mCPP), which has some preference for the 5-HT_{2c} receptor, reduces food intake in mice that express the normal 5-HT_{2c} receptor while the compound lacks activity in mice expressing the mutated inactive form of the 5-HT_{2c} receptor (Nature 1995, 374, 542-546). In a recent clinical study, a slight but sustained reduction in body weight was obtained after 2 weeks of treatment with mCPP in obese subjects (Psychopharmacology 1997, 133, 309-312). Recently, a series of pyrrolo[3,2,1-ij]quinoline derivatives was identified to be 5-HT_{2C} receptor agonists having selectivity over the 5-HT2A receptor (Isaac M., et al., Bioorg. Med. Chem. Lett. 2000, 10, 919-921). The compounds are said to offer a novel approach to the treatment of obesity and epilepsy.

Weight reduction has also been reported from clinical studies with other "serotonergic" agents (see e.g. IDrugs 1998, 1, 456-470). For example, the 5-HT reuptake inhibitor fluoxetine and the 5-HT releasing agent/reuptake inhibitor dexfenfluramine have exhibited weight reduction in controlled studies. However, currently available drugs that increase serotonergic transmission appear to have only a moderate and, in some cases, transient effects on the body weight.

The 5-HT_{2c} receptor subtype has also been suggested to be involved in CNS disorders such as depression and anxiety (Exp. Opin. Invest. Drugs 1998, 7, 1587-1599; IDrugs, 1999, 2, 109-120).

The 5-HT_{2c} receptor subtype has further been suggested to be involved in urinary disorders such as urinary incontinence (IDrugs, 1999, 2, 109-120).

Compounds which have a selective effect on the 5-HT_{2c} receptor may therefore have a therapeutic potential in the treatment of disorders like those mentioned above. Of course, selectivity also reduces the potential for adverse effects mediated by other serotonin receptors.

### Information Disclosure

US-A-3,253,989 discloses the use of mCPP as an anorectic agent.

EP-A1-863 136 discloses azetidine and pyrrolidine derivatives which are selective 5-HT_{2c} receptor agonists having antidepressant activity and which can be used for treating or preventing serotonin-related diseases, including eating disorders and anxiety.

EP-A1-330 263 discloses piperazinylalkylpyrimidines as hypoglycemic agents.

WO 87/04928 discloses 2-(1-piperazinyl)pyrimidines as agents for treating neuropathy.

EP-A2-226842 discloses 1,4-naphthalenedione heterocyclic derivatives as antiallergics and antiasthmatics including 2-(3-bromophenyl)-4-(1-piperazinyl)-pyrimidine.

EP-A-657 426 discloses tricyclic pyrrole derivatives having activity on the 5-HT_{2c} receptor and which inter alia may be used for treating eating disorders.

EP-A-655 440 discloses 1-aminoethylindoles having activity on the 5-HT_{2c} receptor and which may be used for treating eating disorders.

EP-A-572 863 discloses pyrazinoindoles having activity on the 5-HT_{2c} receptor and which may be used for treating eating disorders.

J. Med. Chem. 1978, 21, 536-542 and US-A-4,081,542 disclose a series of piperazinylpyrazines having central serotonin-mimetic activity.

US 4,078,063 discloses a series of piperazinylpyridines having anorexic activity.

J. Med. Chem. 1981, 24, 93-101 discloses a series of piperazinylquinoxalines with central serotoninmimetic activity.

ES 514549 discloses piperazine derivative with anorexigenic action.

EP 370560 discloses 1-[mono- or bis(trifluoromethyl)-2-pyridinyl]piperazines as central nervous system agents.

J. Med. Chem. 1987, 30, 1794-1798 discloses 2-(4-heterocyclylpiperazin-1-yl) derivatives including 2-phenoxy-4-piperazin-1-ylpyrimidine.

DE 2202385 discloses antimicrobial (5-nitro-2-furyl)pyrimidines and - thiadiazoles including 2-(5-nitro-2-furyl)-4-(4-methyl-1-piperazinyl)pyrimidine and 2-(5-nitro-2-furyl)-4-[4-(2-hydroxyethyl)-1-piperazinyl]pyrimidine.

J. Med Chem. 1987, 30, 1210-1214 discloses *N*,*N*-disubstituted 6-alkoxy-2-pyridinamines as anticonvulsant agents including 1-(6-methoxy-2-pyridinyl)piperazine, 1-(6-ethoxy-2-pyridinyl)piperazine, 1-(6-isopropoxy-2-pyridinyl)piperazine, 1-(6-isobutoxy-2-pyridinyl)piperazine, 1-(6-cyclopropylmethoxy-2-pyridinyl)piperazine, 1-(6-cyclohexylmethoxy-2-pyridinyl)piperazine, and 1-(6-cyclohexyloxy-2-pyridinyl)piperazine.

J. Med. Chem. 1989, 32, 1237-1242 discloses 6-alkyl-*N*,*N*-disubstituted-2-pyridinamines as anticonvulsant agents including 1-(6-butylthio-2-pyridinyl)piperazine, 1-(6-cyclohexylmethyl-2-pyridinyl)piperazine and 1-[6-(2-phenylethyl)-2-pyridinyl]piperazine.

JP 07300474 discloses drugs for treatment of diseases related to serotoninergic nerve including 1-(6-phenoxy-2-pyridinyl)piperazine and 1-[6-(substituted)phenoxy-2-pyridinyl]piperazines, 1-(6-benzyloxy-2-pyridinyl)piperazine, 1-(6-cyclobutyloxy-2-pyridinyl)piperazine, and 1-(6-cyclopentyloxy-2-pyridinyl)piperazine

EP 580465 discloses heterocyclic piperazines as 5-HT₃ agonists including 6-chloro-2-(3-methylpiperazinyl)pyridine and 6-chloro-2-(4-methylpiperazinyl)pyridine.

WO 00/12475 discloses indoline derivatives as 5-HT_{2b} and/or 5-HT_{2c} receptor ligands, especially for the treatment of obesity.

WO 00/12510 discloses pyrroloindoles, pyridoindoles and azepinoindoles as 5-HT_{2c} receptor agonists, particluarly for the treatment of obesity.

WO 00/12482 discloses indazole derivatives as selective, directly active 5-HT_{2c} receptor ligands, preferably 5-HT_{2c} receptor agonists, particularly for use as anti-obesity agents.

WO 00/12502 discloses pyrroloquinolines as 5-HT_{2c} receptor agonists, particularly for use as anti-obesity agents.

WO 00/35922 discloses 2,3,4,4*a*-tetrahydro-1*H*-pyrazino[1,2-*a*]quinoxalin-5(6*H*)ones as 5HT_{2c} agonists, which may be used for the treatment of obesity.

WO 00/44737 discloses aminoalkylbenzofurans as 5-HT_{2c} agonists, which may be used for the treatment of obesity.

Further compounds reported to be 5HT_{2c} receptor agonists are, for example, indazolylpropylamines of the type described in WO 00/12481; indazoles of the type described in WO 00/17170; piperazinylpyrazines of the type described in WO 00/76984; heterocycle fused γ-carbolines of the type described in WO 00/77001, WO 00/77002 and WO 00/77010; benzofurylpiperazines of the type described in WO 01/09111 and WO 01/09123; benzofurans of the type described in WO 01/09122; benzothiophenes of the type described in 01/09126; aminoalkylindazoles of the type described in WO 98/30548; indoles of the type described in WO 01/12603; indolines of the type described in WO 01/12602; pyrazino(aza)indoles of the type described in WO 00/44753 and tricyclic pyrroles or pyrazoles of the type described in WO 98/56768.

WO 96/11920 discloses CNS-active pyridinylurea derivatives.

WO 95/01976 discloses indoline derivatives active as 5-HT_{2c} antagonists and of potential use in the treatment of CNS disorders.

WO 99/58490 disloses aryl-hydronaphthalen-alkanamines which may effectuate partial or complete blockage of serotonergic 5-HT_{2c} receptors in an organism.

### Summary of the Invention

According to the invention novel compounds of the general formula (II) are provided: 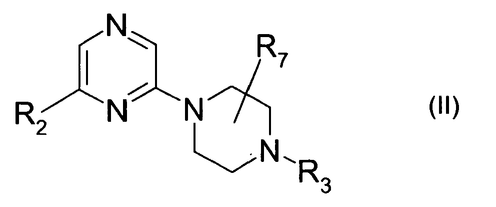 wherein
R₂ is selected from the group consisting of aryl-C₁-C₆-alkyl, aryl-C₁-C₆-alkoxy, heteroaryl-C₁-C₆-alkoxy, aryloxy-C₂-C₆-alkoxy, heteroaryloxy-C₂-C₆-alkoxy, 1-indanyloxy, 2-indanyloxy, aryloxy, heteroaryloxy, arylthio, heteroarylthio, C₅-C₆-cycloalkylthio, C₅-C₈-alkoxy, C₅-C₈-alkylthio, C₃-C₆-alkynyloxy, C₃-C₆-alkenyloxy, fluoro-C₂-C₄-alkoxy, C₄-C₈-cycloalkyloxy, C₃-C₈-cycloalkyl-C₁-C₄-alkoxy, halogen, aryl-C₁-C₄-alkylthio, heteroaryl-C₁-C₄-alkylthio, aryl-C₁-C₄-alkylamino, heteroaryl-C₁-C₄-alkylamino, heteroaryl and aryl;
with the proviso that:
(i) R₂ is other than phenylthio, phenylmethylthio, phenyl or phenyl substituted by halogen;
(ii) R₂ in formula (II) is halogen only when the piperazine ring is 2-methylpiperazin-1-yl or 2-ethylpiperazin-1-yl;
and wherein any aryl or heteroaryl residue, alone or as part of another group, in R₂ may be independently substituted in one or more positions, preferably one or two, by C₁₋₄-alkyl, C₁₋₄-alkoxy, C₁₋₄-alkylthio, C₂₋₄-acyl, C₁₋₄-alkylsulphonyl, cyano, nitro, hydroxy, C₂₋₆-alkenyl, C₂₋₆-alkynyl, fluoromethyl, trifluoromethyl, trifluoromethoxy, halogen, -N(R₅)(R₆), aryl, aryloxy, arylthio, aryl-C₁₋₄-alkyl, aryl-C₂₋₄-alkenyl, aryl-C₂₋₄-alkynyl, heteroaryl, heteroaryloxy, heteroarylthio or heteroaryl-C₁₋₄-alkyl, aryl-C₁₋₄-alkoxy, aryloxy-C₁₋₄-alkyl, dimethylamino-C₂₋₄-alkoxy;
and wherein any aryl or heteroaryl residue as substituents on aryl or heteroaryl, alone or as part of another group, in R₂ in turn may be substituted in one or more postions, preferably one, independently of each other by C₁₋₄-alkyl, C₁₋₄-alkoxy, halogen, trifluoromethyl, cyano, hydroxy or dimethylamino;
R₃ is H or C₁₋₄-alkyl, al lyl, 2-hydroxyethyl or 2-cyanoethyl, preferably hydrogen;
R₅ and R₆ independently of each other are hydrogen, methyl or ethyl, or together with the nitrogen atom to which they are bound form a pyrrolidine, piperazine, morpholine, thiomorpholine or a piperidine ring;
R₇ is hydrogen or C₁₋₄ alkyl, preferably hydrogen, methyl or ethyl, more preferably hydrogen or methyl;
and pharmaceutically acceptable salts, hydrates, geometrical isomers, tautomers, optical isomers and *N*-oxides thereof.

In case the compounds of formula (II) can be in the form of optical isomers, the invention comprises the racemic mixture as well as the individual enantiomers as such.

In case the compounds of formula (II) contain groups, which may exist in tautomeric forms, the invention comprises the tautomeric forms of the compounds as well as mixtures thereof.

In case the compounds of formula (II) can be in the form of geometrical isomers, the invention comprises the geometrical isomers as well as mixtures thereof.

According to another aspect, the invention provides the compounds according to formula (II) above for use in therapy.

Still another aspect of the invention provides a pharmaceutical composition comprising a compound according to formula (II) above as the active ingredient, preferably together with a pharmaceutically acceptable carrier and, if desired, other pharmacologically active agents.

Another aspect of the invention provides for the use of the compounds according to formula (II) above for the manufacture of a medicament for the treatment of a serotonin-related disease, particularly 5-HT_{2c} receptor-related, especially eating disorders, particularly obesity; memory disorders; schizophrenia, mood disorders, anxiety disorders, pain, substance abuse, sexual dysfunctions, epilepsy and urinary disorders.

### Detailed Description of the Invention

According to the present invention, a class of novel compounds has been developed which compounds bind to the 5-HT_{2c} receptor (agonists and antagonists) and which therefore may be used for the treatment of serotonin-related disorders.

First, the various terms used, separately and in combinations, in the above definition of the compounds having the general formula (II) will be explained.

By "heteroatom" is meant nitrogen, oxygen, sulphur, and in heterocyclic rings (including heteroaromatic as well as saturated and partially saturated heterocyclic rings), also selenium.

The term "aryl" is intended to include aromatic rings (monocyclic or bicyclic) having from 6 to 10 ring carbon atoms, such as phenyl, 1-naphthyl, 2-naphthyl, 1,2,3,4-tetrahydronaphthyl (can be linked to the remainder of the molecule via a carbon atom in any ring) and indanyl (can be linked to the remainder of the molecule via a carbon atom in any ring).

The term "heteroaryl" means a mono- or bicyclic aromatic ring system, only one ring need be aromatic, and which can be linked to the remainder of the molecule via a carbon or nitrogen atom in any ring, and having from 5 to 10 ring atoms (mono- or bicyclic), in which one or more of the ring atoms are other than carbon, such as nitrogen, sulphur, oxygen and selenium. Examples of such heteroaryl rings are pyrrole, imidazole, thiophene, furan, thiazole, isothiazole, thiadiazole, oxazole, isoxazole, oxadiazole, pyridine, pyrazine, pyrimidine, pyridazine, pyrazole, triazole, tetrazole, chroman, isochroman, coumarin, quinoline, quinoxaline, isoquinoline, phthalazine, cinnoline, quinazoline, indole, isoindole, indoline, isoindoline, benzothiophene, benzofuran, 2,3-dihydrobenzofuran, isobenzofuran, benzoxazole, 2,1,3-benzoxadiazole, benzothiazole, 2,1,3-benzothiadiazole, 2,1,3-benzoselenadiazole, benzimidazole, indazole, 2,3-dihydro-1,4-benzodioxine, 1,3-benzodioxole, 1,2,3,4-tetrahydroquinoline, 3,4-dihydro-2*H*-1,4-benzoxazine, 1,5-naphthyridine, 1,8-naphthyridine, 3,4-dihydro-2*H*-pyrido[3,2-b]-1,4-oxazine, and 2,3-dihydro-1,4-benzoxathiine. If a bicyclic aryl or heteroaryl ring is substituted, it may be substituted in any ring.

Exemplary aryl-C₁-C₆-alkyl, in which the alkyl portion of the group may be straight or branched, include benzyl, 2-phenylethyl, 3-phenyl-1-propyl, 1-phenylethyl, 1-phenyl-2-propyl and the like.

Exemplary aryl-C₁-C₆-alkoxy, in which the alkyl portion of the group may be straight or branched, include benzyloxy, 2-naphthylmethoxy, 2-phenylethoxy, 3-phenyl-1-propoxy, 1-phenylethoxy, 1-phenyl-2-propoxy, 2-phenyl-1-propoxy and the like.

Exemplary aryloxy-C₂-C₆-alkoxy, in which the alkyl portion of the group may be straight or branched, include 2-phenoxyethoxy, 2-(1-naphthyloxy)ethoxy, 3-(2- naphthyloxy)-1-propoxy, 3-phenoxy-1-propoxy, 4-phenoxy-1-butoxy, 5-phenoxy-1-pentoxy, 1-phenoxy-2-propoxy and the like.

Exemplary C₃-C₈-cycloalkyl-C₁-C₄-alkoxy, in which the alkyl portion of the group may be straight or branched, include cyclopropylmethoxy, cyclopentylmethoxy, 2-cyclohexylethoxy, 1-cyclohexylethoxy, 1-cyclopropylethoxy, 1-cyclobutylethoxy and the like.

Exemplary heteroaryl-C₁-C₄-alkylamino include 2-(2-pyridinyl)ethylamino, 3-pyridinylmethylamino, 2-(2-thienyl)ethylamino, 2-(1*H*-indol-3-yl)ethylamino and the like.

Exemplary heteroaryloxy-C₂-C₆-alkoxy include 2-(8-quinolinyloxy)ethoxy, 2-(3-pyridinyloxy)ethoxy, 3-(8-quinolinyloxy)propoxy and the like

Exemplary C₃-C₆-alkynytoxy include propargyloxy, 1-hexynyloxy, 2-hexynyloxy, 3-butynyloxy, 3-pentynyloxy and the like.

C₅₋₈-alkoxy may be straight or branched. Exemplary alkoxy groups include pentyloxy, isopentyloxy, hexyloxy, and isohexyloxy.

Halogen includes fluorine, chlorine or bromine.

Where it is stated above that aryl and heteroaryl residues may be substituted (in one or more postions), this applies to aryl and heteroaryl *per se* as well as to any combined groups containing aryl or heteroaryl residues, such as heteroaryloxy-C₂-C₆-alkoxy, heteroaryloxy, aryl-C₁-C₆-alkoxy etc.

The term "*N*-oxides" means that one or more nitrogen atoms, when present in a compound, are in *N*-oxide form (N→O).

The term "prodrug forms" means a pharmacologically acceptable derivative, such as a carbamate or an amide, which derivative is biotransformed in the body to form the active drug. Reference is made to Goodman and Gilman's, The Pharmacological basis of Therapeutics, 8th ed., McGraw-Hill, Int. Ed. 1992, "Biotransformation of Drugs, p. 13-15.

"Pharmaceutically acceptable" means being useful in preparing a pharmaceutical composition that is generally safe, non-toxic and neither biologically nor otherwise undesirable and includes being useful for veterinary use as well as human pharmaceutical use.

"Pharmaceutically acceptable salts" mean salts which are pharmaceutically acceptable, as defined above, and which possess the desired pharmacological activity. Such salts include acid addition salts formed with organic and inorganic acids, such as hydrogen chloride, hydrogen bromide, hydrogen iodide, sulfuric acid, phosphoric acid, acetic acid, glycolic acid, maleic acid, malonic acid, oxalic acid, toluenesulphonic acid, methanesulphonic acid, fumaric acid, succinic acid, tartaric acid, citric acid, benzoic acid, ascorbic acid and the like.

In formula (II), R₃ is preferably hydrogen, and R₇ is preferably hydrogen or C₁₋₄-alkyl. When R₇ is C₁₋₄-alkyl, it is most preferably substituted in the 2-postion of the piperazine ring. R₇ is most preferably hydrogen or methyl.

Preferred compounds of the general formula (II) above are:
2-(Benzyloxy)-6-(1-piperazinyl)pyrazine,
2-[(2-Methoxybenzyl)oxy]-6-(1-piperazinyl)pyrazine,
2-[(3-Methoxybenzyl)oxy]-6-(1-piperazinyl)pyrazine,
2-[(3,5-Difluorobenzyl)oxy]-6-(1-piperazinyl)pyrazine,
2-(1-Naphthylmethoxy)-6-(1-piperazinyl)pyrazine,
2-(1-Phenylethoxy)-6-(1-piperazinyl)pyrazine,
2-[1-(3-Fluorophenyl)ethoxy]-6-(1-piperazinyl)pyrazine,
2-[1-(2-Methoxyphenyl)ethoxy]-6-(1-piperazinyl)pyrazine,
2-(3,4-Dihydro-2*H*-chromen-4-yloxy)-6-(1-piperazinyl)pyrazine,
2-(2-Phenylethoxy)-6-(1-piperazinyl)pyrazine,
2-[(2-Phenoxybenzyl)oxy]-6-(1-piperazinyl)pyrazine,
2-[2-(3-Chlorophenyl)ethoxy]-6-(1-piperazinyl)pyrazine,
2-[2-(2-Methoxyphenyl)ethoxy]-6-(1-piperazinyl)pyrazine,
2-[2-(3-Methoxyphenyl)ethoxy]-6-(1-piperazinyl)pyrazine,
2-[2-(4-Methoxyphenyl)ethoxy]-6-(1-piperazinyl)pyrazine,
2-[2-(2,5-Dimethoxyphenyl)ethoxy]-6-(1-piperazinyl)pyrazine,
2-[(2-Phenylethyl)sulfanyl]-6-(1-piperazinyl)pyrazine,
2-[(5-Fluoro-2-methoxybenzyl)oxy]-6-(1-piperazinyl)pyrazine,
2-[(3-Cyanobenzyl)oxy]-6-(1-piperazinyl)pyrazine,
2-[(2-Chlorobenzyl)sulfanyl]-6-(1-piperazinyl)pyrazine,
2-[2-(4-Dimethylaminophenyl)ethoxy]-6-(1-piperazinyl)pyrazine,
2-[2-(1*H*-Indol-3-yl)ethoxy]-6-(1-piperazinyl)pyrazine,
2-[2-(1*H*-Indol-1-yl)ethoxy]-6-(1-piperazinyl)pyrazine,
2-Benzyl-6-(1-piperazinyl)pyrazine,
2-[(3,5-Dimethoxybenzyl)oxy]-6-(1-piperazinyl)pyrazine,
1-[6-(Benzyloxy)-2-pyrazinyl]-2-methylpiperazine,
1-[6-(Benzyloxy)-2-pyrazinyl]-2-ethylpiperazine
2-[2-(2-Fluorophenyl)ethoxy]-6-(1-piperazinyl)pyrazine,
2-(2,3-Dihydro-1*H*-inden-1-ylmethoxy)-6-(1-piperazinyl)pyrazine
2-(4-Phenoxybutoxy)-6-(1-piperazinyl)pyrazine.
2-[(5-Phenoxypentyl)oxy]-6-(1-piperazinyl)pyrazine.
2-[(2,5-Dimethoxybenzyl)oxy]-6-(1-piperazinyl)pyrazine.
2- {[2-(2-Phenytethyl)benzyl]oxy}-6-(1-piperazinyl)pyrazine
(2R)-1-[6-(Benzyloxy)-2-pyrazinyl]-2-methylpiperazine,
2-[2-(2,6-Difluorophenoxy)ethoxy]-6-(1-piperazinyl)pyrazine
2-[2-(2-Naphthyloxy)ethoxy]-6-(1-piperazinyl)pyrazine
2-(1-Methyl-2-phenylethoxy)-6-(1-piperazinyl)pyrazine
2-{[2-(Phenoxymethyl)benzyl]oxy}-6-(1-piperazinyl)pyrazine
2-[(5-Fluoro-2-methoxybenzyl)oxy]-6-(1-piperazinyl)pyrazine
2-[(2,5-Difluorobenzyl)oxy]-6-(1-piperazinyl)pyrazine
2-[(2-Fluorobenzyl)oxy]-6-(1-piperazinyl)pyrazine
2-(Benzo[*b*]thiophen-3-ylmethoxy)-6-(1-piperazinyl)pyrazine,
2-[2-(5-Methyl-2-phenyl-oxazol-4-yl)-ethoxy]-6-(1-piperazinyl)pyrazine,
2-[1-(2,6-Difluoro-phenyl)-ethoxy]-6-(1-piperazinyl)pyrazine,
2-(2-Naphthalen-2-yl-ethoxy)-6-(1-piperazinyl)pyrazine,
2-[3-(Naphthalen-2-yloxy)-propoxy]-6-(1-piperazinyl)pyrazine,
2-[2-(7-Methoxy-naphthalen-2-yloxy)-ethoxy]-6-(1-piperazinyl)pyrazine,
2-[5-(4-Chlorophenyl)-2-methylfuran-3-ylmethoxy]-6-(1-piperazinyl)pyrazine,
2-(1*H*-Indol-4-ylmethoxy)-6-(1-piperazinyl)pyrazine,
2- {[3-(Benzyloxy)benzyl]oxy} -6-(1-piperazinyl)pyrazine,
and their pharmacologically acceptable salts and solvates.

As mentioned above, the compounds of the present invention are useful for the treatment (including prophylactic treatment) of serotonin-related disorders, especially 5-HT_{2c} receptor-related, in a human being or in an animal (including e.g. pets), such as eating disorders, especially obesity; memory disorders, such as Alzheimer's disease; schizophrenia; mood disorders, including, but not restricted to, major depression and bipolar depression, including both mild and manic bipolar disorder, seasonal affective disorder (SAD); anxiety disorders, including situational anxiety, generalized anxiety disorder, primary anxiety disorders (panic disorders, phobias, obsessive-compulsive disorders, and post-traumatic stress disorders), and secondary anxiety disorders (for example anxiety associated with substance abuse); pain; substance abuse; sexual dysfunctions; epilepsy; and urinary disorders, such as urinary incontinence. Additionally, the compounds of the present invention are generally useful in treatment of diseases and disorders of the central nervous system (CNS).

The compounds of the present invention in radiolabelled form, may be used as a diagnostic agent.

The compounds of general formula (II) above may be prepared by, or in analogy with, conventional methods.

An obtained compound of formula (II) may be converted to another compound of formula (II) by methods well known in the art.

The processes described above may be carried out to give a compound of the invention in the form of a free base or as an acid addition salt. A pharmaceutically acceptable acid addition salt may be obtained by dissolving the free base in a suitable organic solvent and treating the solution with an acid, in accordance with conventional procedures for preparing acid addition salts from base compounds. Examples of addition salt forming acids are maleic acid, fumaric acid, succinic acid, methanesulfonic acid, acetic acid, oxalic acid, benzoic acid, hydrochloric acid, sulphuric acid, phosphoric acid, and the like.

The compounds of formula (II) may possess one or more chiral carbon atoms, and they may therefore be obtained in the form of optical isomers, e.g. as a pure enantiomer, or as a mixture of enantiomers (racemate) or as a mixture containing diastereomers. The separation of mixtures of optical isomers to obtain pure enantiomers is well known in the art and may, for example, be achieved by fractional crystallization of salts with optically active (chiral) acids or by chromatographic separation on chiral columns.

The necessary starting materials for preparing the compounds of formula (II) are either known or may be prepared in analogy with the preparation of known compounds.

In accordance with the present invention, the compounds of formula (II), in the form of free bases or salts with physiologically acceptable acids, can be brought into suitable galenic forms, such as compositions for oral use, for injection, for nasal spray administration or the like, in accordance with accepted pharmaceutical procedures. Such pharmaceutical compositions according to the invention comprise an effective amount of the compounds of formula (II) in association with compatible pharmaceutically acceptable carrier materials, or diluents, as are well known in the art. The carriers may be any inert material, organic or inorganic, suitable for enteral, percutaneous, subcutaneous or parenteral administration, such as: water, gelatin, gum arabicum, lactose, microcrystalline cellulose, starch, sodium starch glycolate, calcium hydrogen phosphate, magnesium stearate, talcum, colloidal silicon dioxide, and the like. Such compositions may also contain other pharmacologically active agents, and conventional additives, such as stabilizers, wetting agents, emulsifiers, flavouring agents, buffers, and the like.

The compositions according to the invention can e.g. be made up in solid or liquid form for oral administration, such as tablets, pills, capsules, powders, syrups, elixirs, dispersable granules, cachets, suppositories and the like, in the form of sterile solutions, suspensions or emulsions for parenteral administration, sprays, e.g. a nasal spray, transdermal preparations, e.g. patches, and the like.

As mentioned above, the compounds of the invention may be used for the treatment of serotonin-related disorders in a human being or an animal, such as eating disorders, particularly obesity, memory disorders, schizophrenia, mood disorders, anxiety disorders, pain, substance abuse, sexual dysfunctions, epilepsy, and urinary disorders. The dose level and frequency of dosage of the specific compound will vary depending on a variety of factors including the potency of the specific compound employed, the metabolic stability and length of action of that compound, the patient's age, body weight, general health, sex, diet, mode and time of administration, rate of excretion, drug combination, the severity of the condition to be treated, and the patient undergoing therapy. The daily dosage may, for example, range from about 0.001 mg to about 100 mg per kilo of body weight, administered singly or multiply in doses, e.g. from about 0.01 mg to about 25 mg each. Normally, such a dosage is given orally but parenteral administration may also be chosen.

The invention will now be illustrated with the following examples, which however, are for illustrative purposes are not intended to limit the scope of the invention.

### EXAMPLES

### General:

The structures of the prepared compounds were confirmed by standard spectroscopical methods, and elemental analysis and/or high resolution MS. The NMR data were obtained on a JEOL JNM-EX 270, a Bruker 400 DPX or a Bruker DRX 500 spectrometer. IR spectra were obtained on a Perkin Elmer SPECTRUM 1000 FT-IR spectrometer. High resolution MS was obtained on a Micromass LCT spectrometer. Elemental analysis was performed by Mikro Kemi AB, Uppsala, Sweden or at Pharmacia AB, Stockholm, Sweden. Melting points, when given, were obtained on a Büchi or a Gallenkamp melting point apparatus and are uncorrected.

### EXAMPLE 1

### 2-(1-Naphthylmethoxy)-6-(1-piperazinyl)pyrazine.

To a solution of 2,6-dichloropyrazine (298 mg, 2.00 mmol) and 1-naphthylmethanol (348 mg, 2.20 mmol) in dioxane (5 mL) was added NaH (55 % in mineral oil, 96 mg, 2.2 mmol) at room temperature. The reaction was stirred at room temperature and monitored by GC. After 3 h, piperazine (189 mg, 2.20 mmol) and NaH (55% in oil, 96 mg, 2.2 mmol) was added into the reaction flask at room temperature. The reaction mixture was stirred at room temperature for 24 h. The solvent was evaporated off. To the residue was added piperazine (671 mg, 7.80 mmol) and acetonitrile (5 mL) and the solution was heated under reflux for 5 h. The reaction mixture was directly loaded on a short column of silica gel for flash column chromatography. Elution with MeOH/dichloromethane (1:9) furnished 0.41 g (64%) of the title compound. HRMS *m*/*z* calcd for C₁₉H₂₀N₄O (M)⁺ 321.1715, found 321.1721. Anal. (C₁₉H₂₀N₄O ·0.1 H₂O) C, H, N.

### EXAMPLE 2

### 2-[1-(3-Fluorophenyl)ethoxy]-6-(1-piperazinyl)pyrazine.

### Step 1: 2-Chloro-6-[1-(3-fluorophenyl)ethoxy]pyrazine.

To a solution of 2,6-dichloropyrazine (298 mg, 2.00 mmol) and 1-(3-fluorophenyl)ethanol (308 mg, 2.2 mmol) in dioxane (5 mL) was added NaH (55 % in oil, 96 mg, 2.2 mmol) at room temperature. The reaction mixture was stirred over night. Water (0.5 mL) was added and the mixture was stirred for 15 min. Drying (K₂CO₃), filtration, and concentration *in vacuo* gave the title compound as an oil (0.55 g) that was used directly in the next step. MS *m*/*z* 254 (M+H)⁺.

### Step 2: 2-[1-(3-Fluorophenyl)ethoxy]-6-(1-piperazinyl)pyrazine.

A mixture of the product from step 1 above (crude, 1.09 g, ~ 4.3 mmol), piperazine (1.03 g, 12.0 mmol) and K₂CO₃ (1.00 g, 7.2 mmol) in acetonitrile (5 mL) was heated under reflux overnight (20 h). After cooling, ethyl acetate (15 mL) and water (5 mL) were added. The ethyl acetate layer was filtered through a short column of silica gel using MeOH/ethyl ether (1:1) as eluent to give 0.72 g (55%) of the title compound as an oil. HRMS *m*/*z* calcd for C₁₆H₁₉N₄O₄F (M)⁺ 302.1543, found 302.1528. Anal. (C₁₆H₁₉N₄OF 0.5 H₂O) C, H, N.

### EXAMPLE 3

### 2-(1,3-Benzodioxol-5-ylmethoxy)-6-(1-piperazinyl)pyrazine, Acetate.

### Step 1: 2-(1,3-Benzodioxol-5-ylmethoxy)-6-chloropyrazine.

To a solution of 2,6-dichloropyrazine (298 mg, 2.00 mmol) and piperonyl alcohol (335 mg, 2.20 mmol) in dioxane (5 mL) was added NaH (55 % in mineral oil, 96 mg, 2.2 mmol) at room temperature. The reaction mixture was stirred overnight. Water (0.5 mL) was added and the mixture was stirred for 15 min. Drying, Na₂CO₃, filtration and concentration *in vacuo* furnished an oil (0.54 g) that was used directly in the next step. HRMS *m*/*z* calcd for C₁₂H₉ClN₂O₃ (M)⁺ 264.0302, found 264.0303.

### Step 2: 2-(1,3-Benzodioxol-5-ylmethoxy)-6-(1-piperazinyl)pyrazine, Acetate.

A mixture of the product from step 1 above (0.54 g, 2.0 mmol), piperazine (0.86 g, 10 mmol) and K₂CO₃ (1.00 g, 7.2 mmol) in acetonitrile (5 mL) was heated under reflux for 5 h. After cooling, ethyl acetate (20 mL) and water (5 mL) were added. The saved ethyl acetate layer was filtered through a short column of silica gel using methanol/ethyl ether (1:1) as eluent. This furnished the free base of the title compound as an oil (0.43 g). This material was dissolved in methanol and acetic acid (0.5 mL) was added. The solution was concentrated. Diethyl ether (25 mL) was added and the flask was shaken until crystallization started. The crystals was collected, washed with diethyl ether and dried in air to give 0.34 g (45%) of the title compound: mp 124-127 °C. HRMS *m*/*z* calcd for C₁₆H₁₈N₄O₃ (M)⁺ 314.1379, found 314.1308. Anal. (C₁₆H₁₈N₄O₃ - CH₃COOH - 1.6H₂O) C, H, N.

### EXAMPLE 4

### 2-[(3-Methoxybenzyl)oxy]-6-(1-piperazinyl)pyrazine, Acetate.

### Step 1: 2-Chloro-6-[(3-methoxybenzyl)oxy]pyrazine.

To a solution of 2,6-dichloropyrazine (444 mg, 3.00 mmol) and 3-methoxybenzyl alcohol (455 mg, 3.30 mmol) in dioxane (5 mL) was added NaH (55 % in oil, 144 mg, 3.30 mmol) at room temperature. The reaction mixture was stirred overnight. Water (0.5 mL) and ethyl acetate (10 mL) were added and the mixture was stirred for 15 min and then filtered. The filtrate was dried over K₂CO₃ and concentrated to give an oil (0.86 g) that was used directly in the next step. MS *m*/*z* 250 (M+H)⁺.

### Step 2: 2-[(3-Methoxybenzyl)oxy]-6-(1-piperazinyl)pyrazine, Acetate.

A mixture of the product from step I above (0.57 g, ~ 2.0 mmol), piperazine (0.86 g, 10 mmol) and K₂CO₃ (1.00 g, 7.2 mmol) in acetonitrile (5 mL) was heated under reflux for 10 h. After cooling, ethyl acetate (30 mL) was added. The ethyl acetate layer was washed with water and brine, dried over Na₂CO₃, and concentrated under reduced pressure. The residue was dissolved in diethyl ether. Acetic acid (0.5 mL) was added and the solution was left at room temperature for crystallization. The crystals were collected, washed with diethyl ether and dried in vaccum to give 0.54 g (75%) of the title compound: mp 111-113 °C. HRMS *m*/*z* calcd for C₁₆H₂₀N₄O₂ (M)⁺ 300.1586, found 300.1589. Anal. (C₁₆H₂₀N₄O₂ · CH₃COOH) C, H, N.

### EXAMPLE 5

### 2-[(2-Methoxybenzyl)oxy]-6-(1-piperazinyl)pyrazine, Dihydrochloride.

### Step 1: 2-Chloro-6-[(2-methoxybenzyl)oxy]pyrazine.

The title compound was prepared according to the procedure of example 4, step I starting from 2,6-dichloropyrazine (298 mg, 2.00 mmol), 2-methoxybenzyl alcohol (303 mg, 2.20 mmol) and NaH (55 % in mineral oil, 96 mg, 2.2 mmol). The yield of the crude product was 0.49 g (98%) and was used directly as such in the next step. MS *m*/*z* 250 (M)⁺. HRMS *m*/*z* calcd for C₁₂H₁₁ ClN₂O₂ (M)⁺ 250.0509, found 250.0522.

### Step 2: 2-[(2-Methoxybenzyl)oxy]-6-(1-piperazinyl)pyrazine, Dihydrochloride.

The title compound was prepared according to the procedure of example 4, step 2 starting from the product of step 1 above (0.49 g), piperazine (0.86 g, 10 mmol) and K₂CO₃ (1.00 g, 7.2 mmol). This gave the free base of the title compound as an oil. Yield 0.43 g (73%). The free base was dissolved in ethyl ether and a solution of HCl in diethyl ether was added until no more precipitate was formed. The precipitate was collected, washed with diethyl ether, and dried in vaccum to give 0.41 g (56%) of the title compound: mp 171-173 °C). HRMS *m*/*z* calcd for C₁₆H₂₀N₄O₂ (M)⁺ 300.1586, found 300.1586. Anal. (C₁₆H₂₀N₄O₂ · 2HCl) C, H, N.

### EXAMPLE 6

### 2-[(3,5-Difluorobenzyl)oxy]-6-(1-piperazinyl)pyrazine, Acetate.

### Step 1: 2-Chloro-6-[(3,5-difluorobenzyl)oxy]pyrazine.

The title compound was prepared according to the procedure of example 4, step 1 starting from 2,6-dichloropyrazine (444 mg, 3.00 mmol), 3,5-difluorobenzyl alcohol (475 mg, 3.30 mmol) and NaH (55% in mineral oil, 144 mg, 3.30 mmol). The solid product was collected, washed with water, and dried to give 0.77 g (100%) of the crude product that was used directly in the next step. An analytical sample was recrystallized from diethyl ether/hexane: mp 70-71 °C. MS *m*/*z* 257 (M+H)⁺. Anal. (C₁₁H₇ClFN₂O) C, H, N.

### Step 2: 2-[(3,5-Difluorobenzyl)oxy]-6-(1-piperazinyl)pyrazine, Acetate.

The title compound was prepared according to the procedure of example 4, step 2 starting from the product of step 1 above (0.51 g, ~ 2.0 mmol), piperazine (0.86 g, 10 mmol) and K₂CO₃ (1.00 g, 7.2 mmol). Yield 0.49 g, (67%); mp 70-72 °C; HRMS *m*/*z* calcd for C₁₅H₁₆F₂N₄O (M)⁺ 306.1292, found 306.1292. Anal. (C₁₅H₁₆F₂N₄O - CH₃COOH·H₂O) C, H, N.

### EXAMPLE 7

### 2-([1,1'-Biphenyl]-4-ylmethoxy)-6-(1-piperazinyl)pyrazine, Acetate.

### Step 1: 2-([1;1'-Biphenyl]-4-ylmethoxy)-6-chloropyrazine.

The title compound was prepared according to the procedure of example 4, step 1 starting from 2,6-dichloropyrazine (444 mg, 3.00 mmol), p-phenylbenzyl alcohol (607 mg, 3.30 mmol) and NaH (55% in mineral oil, 144 mg, 3.30 mmol). Recrystallization from hexane gave 0.55 g (88%) of the title compound: mp 86-87 °C. MS *m*/*z:* 297 (M+H)⁺. Anal. (C₁₇H₁₃ClN₂O) C, H, N.

### Step 2: 2-([1,1'-Biphenyl]-4-ylmethoxy)-6-(1-piperazinyl)pyrazine, Acetate.

The title compound was prepared according to the procedure of example 4, step 2 starting from the product of step 1 above (0.54 g, 1.87 mmol), piperazine (0.86 g, 10.0 mmol) and K₂CO₃ (1.00 g, 7.2 mmol). Yield: 0.35 g (44%); mp 102-104 °C. HRMS *m*/*z* calcd for C₂₁H₂₂N₄O (M)⁺ 346.1794, found 346.1777. Anal. (C₂₁H₂₂N₄O ·CH₃COOH·0.55H₂O) C, H, N.

### EXAMPLE 8

### 2-[2-(3-Chlorophenyl)ethoxy]-6-(1-piperazinyl)pyrazine, Acetate.

### Step 1: 2-Chloro-6-[2-(3-chlorophenyl)ethoxy]pyrazine.

The title compound was prepared according to the procedure of example 4, step 1 starting from 2,6-dichloropyrazine (444 mg, 3.00 mmol) and *m*-chlorophenethyl alcohol (515 mg, 3.30 mmol) and NaH (55% in mineral oil, 144 mg, 3.30 mmol). The crude product (0.92 g) was used directly in the next step. MS *m*/*z* 269 (M+H)⁺.

### Step 2: 2-[2-(3-Chlorophenyl)ethoxy]-6-(1-piperazinyl)pyrazine, Acetate.

The title compound was prepared according to the procedure of example 4, step 2 starting from the product of step 1 above (0.81 g, 3.02 mmol), piperazine (0.86 g, 10 mmol) and K₂CO₃ (1.00 g, 7.2 mmol). Yield: 0.75 g (65%); mp 118-119 °C. HRMS *m*/*z* calcd for C₁₆H₁₉ClN₄O (M)⁺ 318.1247, found 318.1249. Anal. (C₁₆H₁₉ClN₄O· CH₃COOH) C, H, N.

### EXAMPLE 9

### 6-(1-Piperazinyl)-2-pyrazinyl 1,2,3,4-tetrahydro-1-naphthalenyl ether, Acetate.

### Step 1: 2-Chloro-6-(1,2,3,4-tetrahydro-1-naphthalenyloxy)pyrazine.

The title compound was prepared according to the procedure of example 4, step 1 starting from 2,6-dichloropyrazine (444 mg, 3.00 mmol) and 1,2,3,4-tetrahydro-1-naphthol (488 mg, 3.30 mmol) and NaH (55% in oil, 144 mg, 3.30 mmol). The crude product (0.86 g) was used directly in the next step. MS *m*/*z* 261 (M+H)⁺.

### Step 2: 6-(1-Piperazinyl)-2-pyrazinyl 1,2,3,4-tetrahydro-1-naphthalenyl ether, Acetate.

The title compound was prepared according to the procedure of example 4, step 2 starting from the product of step 1 above (0.75 g, 2.88 mmol), piperazine (0.86 g, 10 mmol) and K₂CO₃ (1.00 g, 7.2 mmol). Yield: 0.59 g (55%); mp 160-162 °C. MS *m*/*z* 310 (M)⁺. HRMS m/z calcd for C₁₈H₂₂N₄O (M)⁺ 310.1794, found 310.1799. Anal. (C₁₈H₂₂N₄O·CH₃COOH) C, H, N.

### EXAMPLE 10

### 2-(1-Piperazinyl)-6-{[4-(trifluoromethyl)benzyl]oxy}pyrazine, Acetate.

### Step 1: 2-Chloro-6-{[4-(trifluoromethyl)benzyl]oxy}pyrazine.

The title compound was prepared according to the procedure of example 4, step 1 starting from 2,6-dichloropyrazine (444 mg, 3.00 mmol) and 4-trifluoromethylbenzyl alcohol (581 mg, 3.30 mmol) and NaH (55% in mineral oil, 144 mg, 3.30 mmol). Recrystallization from hexane gave 0.81 g (93%) of the title compound: mp 67-69 °C.
MS *m*/*z* 289 (M+H)⁺. Anal. (C₁₂H₈ClF₃N₂O) C, H, N.

### Step 2: 2-(1-Piperazinyl)-6-{[4-(trifluoromethyl)benzyl]oxy}pyrazine, Acetate.

The title compound was prepared according to the procedure of example 4, step 2 starting from the product of step 1 above (0.54 g, 1.89 mmol), piperazine (0.86 g, 10 mmol) and K₂CO₃ (1.00 g, 7.20 mmol). Yield: 0.36 g (48%); mp 84-85 °C. MS *m*/*z* 338 (M)⁺. HRMS *m*/*z* calcd for C₁₆H₁₇ F₃N₄O (M)⁺ 338.1054, found 338.1063. Anal. (C₁₆H₁₇ClF₃N₄O·CH₃COOH) C, H, N.

### EXAMPLE 11

### 2-(1-Piperazinyl)-6-(3-pyridinylmethoxy)pyrazine, Acetate.

### Step 1: 2-Chloro-6-(3-pyridinylmethoxy)pyrazine.

The title compound was prepared according to the procedure of example 4, step 1 starting from 2,6-dichloropyrazine (444 mg, 3.00 mmol), nicotinic alcohol (360 mg, 3.30 mmol) in dioxane (5 mL) and NaH (55 % in oil, 144 mg, 3.30 mmol). The crude product, obtained as an oil (0.72 g), was used directly in the next step. MS *m*/*z* 221 (M)⁺.

### Step 2: 2-(1-Piperazinyl)-6-(3-pyridinylmethoxy)pyrazine, Acetate.

The title compound was prepared according to the procedure of example 4, step 2 starting from the product of step 1 above (0.74 g, 3.35 mmol), piperazine (0.86 g, 10 mmol) and K₂CO₃ (1.00 g, 7.2 mmol). Yield: 0.73 g (44 %); mp 98-99 °C; MS *m*/*z* 271(M)⁺. HRMS *m*/*z* calcd for C₁₄H₁₇N₅O (M)⁺ 271.1433, found 271.1425. Anal. (C₁₄H₁₇N₅O · CH₃COOH) C, H, N.

### EXAMPLE 12

### 2-(1-Piperazinyl)-6-[2-(3-pyridinyl)ethoxy]pyrazine, Acetate.

### Step 1: 2-Chloro-6-[2-(3-pyridinyl)ethoxy]pyrazine.

The title compound was prepared according to the procedure of example 4, step 1 starting from 2,6-dichloropyrazine (444 mg, 3.00 mmol) and 2-(3-pyridyl)ethanol (405 mg, 3.30 mmol) and NaH (55% in mineral oil, 144 mg, 3.30 mmol). The crude product, obtained as an oil (0.62 g, 88% yield), was used directly in the next step. MS *m*/*z* 235 (M)⁺.

### Step 2: 2-(1-Piperazinyl)-6-[2-(3-pyridinyl)ethoxy]pyrazine, Acetate.

The title compound was prepared according to the procedure of example 4, step 2 starting from the product of step 1 above (0.62 g, 2.64 mmol), piperazine (0.86 g, 10 mmol) and K₂CO₃ (1.00 g, 7.2 mmol). Yield: 0.40 g (44%); mp 90-91 °C. MS *m*/*z* 285 (M)⁺. HRMS *m*/*z* calcd for C₁₅H₁₉N₅O (M)⁺ 285.1590, found 285.1598. Anal. (C₁₅H₁₉N₅O·CH₃COOH) C, H, N.

### EXAMPLE 13

### 2-(2-Furylmethoxy)-6-(1-piperazinyl)pyrazine.

### Step 1: tert-Butyl 4-(6-chloro-2-pyrazinyl)-1-piperazinecarboxylate.

A mixture of *tert*-butyl 1-piperazinecarboxylate (5.07 g, 27.2 mmol), 2,6-dichloropyrazine (3.38 g, 22.7 mmol), and K₂CO₃ (4.09 g, 30.0 mmol) in acetonitrile (20 mL) was stirred at 65 °C for 12.5 h and for a further 15 h at room temperature. Ether was added and the suspension was filtered. Concentration *in vacuo* furnished the crude product as an oil that crystallized upon standing. Purification by chromatography on silica gel using ethyl acetate/*n*-hexane (6:4) as eluent gave 6.1 g (90%) of the title compound as a solid. HRMS *m*/*z* calcd for C₁₃H₁₉ClN₄O₂ (M)⁺ 298.1197, found 298.1211. Anal. (C₁₃H₁₉ClN₄O₂) C, H, N.

### Step 2: 6-Chloro-2-(1-piperazinyl)pyrazine.*

A solution of trifluoroacetic acid (TFA; 6 mL) in dichloromethane (24 mL) was added to a stirred solution of the product of step 1 above (5.79 g, 19.4 mmol) in dichloromethane (20 mL) at 0 °C. After 1 h and 1.5 h of stirring, additional portions (10 mL and 5 mL) of TFA were added. Crushed ice and 5 M aqueous NaOH were added and the mixture was extracted with dichloromethnae (12 x 200 mL). The combined organic layers were dried (K₂CO₃), filtered and concentrated *in vacuo.* This furnished 3.48 g (90%) of the title compound as a light yellow solid.
*Previously described in a) J. Med. Chem. 1978, 21, 536-542; b) US 4,082,844

### Step 3: 2-(2-Furylmethoxy)-6-(1-piperazinyl)pyrazine.

K-*t*-BuO (1.55 g, 13.8 mmol) was added to a mixture of the product from step 2 above (1.40 g, 7.05 mmol) and 2-furanmethanol (5.3 g, 54 mmol). After being stirred for 7.5 h at 110 °C, the mixture was applied onto a bed of silica (16 x 6 cm). Elution with CHCl₃/MeOH (95:5 followed by 90:10) furnished 1.35 g (74%) of the title compound as an oil. HRMS *m*/*z* calcd for C₁₃H₁₆N₄O₂ (M)⁺ 260.1273, found 260.1276. Anal. (C₁₃H₁₆N₄O₂) C, H, N.

### EXAMPLE 14

### 2-(2-Phenylethoxy)-6-(1-piperazinyl)pyrazine, Maleate.

K-*t*-BuO (0.80 g, 7.13 mmol) was added to a mixture of the product from example 13, step 2 (0.638 g, 3.21 mmol) and 2-phenylethanol (5.62 g, 46.0 mmol). After being stirred for 5 h at 105 °C in a sealed flask, the mixture was applied onto a bed of silica (16 x 5 cm). Elution with CHCl₃/MeOH (97:3 followed by 90:10) furnished 0.68 g of an thick beige colored oil. This material was redissolved in ethyl acetate and K₂CO₃ was added. Filtration and concentration *in vacuo* furnished 0.67 g (74%) of the free base of the title compound as an oil. HRMS *m*/*z* calcd for C₁₆H₂₀N₄O (M)⁺ 284.1637, found 284.1630. The free base was converted into its maleate salt which was recrystallized from MeOH/ether: mp 166-168 °C. Anal. (C₁₆H₂₀N₄O· C₄H₄O₄) C, H, N.

### EXAMPLE 15

### 2-(2-Furyl)-6-(1-piperazinyl)pyrazine.

### Step 1: 2-Chloro-6-(2-furyl)pyrazine.

1,2-Dimethoxyethane (130 mL) was added to a mixture of tetrakis(triphenylphosphine)palladium (0) (0.93 g, 0.80 mmol) and 2,6-dichloropyrazine
(2.55 g, 17.1 mmol). After 5 min of stirring at room temperature, furan-2-boronic acid (1.91 g, 17.1 mmol) followed by aqueous Na₂CO₃ (30 mL; 2 M) were added. The mixture was heated at reflux for 1h [TLC monitoring by SiO₂/*n*-hexane/ethyl acetate (90:10)]. The layers were separated and the light brownish water layer was extracted with dichloromethane (2 x 200 mL). The combined organic layers were dried (K₂CO₃), filtered and concentrated *in vacate.* The brown-yellow oil obtained was purified by silica gel chromatography (18.5 x 4 cm) eluting with *n*-hexane/ethyl acetate (90:10). This furnished 1.47 g (48%) of the title compound as a light yellow solid. HRMS *m*/*z* calcd for C₈H₅ClN₂O (M)⁺ 180.0090, found 180.0092. Anal. (C₈H₅ClN₂O) C, H, N.

### Step 2: 2-(2-Furyl)-6-(1-piperazinyl)pyrazine.

A mixture of the product from step 1 above (0.94 g, 5.2 mmol), piperazine (1.28 g, 14.9 mmol, K₂CO₃ (0.87 g, 6.3 mmol) in acetonitrile (5 mL) was heated in a sealed pyrex flask at 85 °C for 3 h. The mixture was diluted with dichloromethane, filtered, and concentrated *in vacuo.* The oily residue was purified by silica gel chromatography (18 x 4 cm) to furnish a yellow oil. This material was redissolved in a small volume of CHCl₃/ether (9:1) and filtered through a short (4 cm) plug of alumina eluting with ether/MeOH (96:4). The filtrate was concentrated *in vacuo* to afford 0.77 g (64%) of the title compound as a light yellow solid. HRMS *m*/*z* calcd for C₁₂H₁₄N₄O (M)⁺ 230.1168, found 230.1170. Anal. (C₁₂H₁₄N₄O) C, H, N.

### EXAMPLE 16

### 2-(1-Piperazinyl)-6-(3-thienyl)pyrazine.

### Step 1: 2-Chloro-6-(3-thienyl)pyrazine.

1,2-Dimethoxyethane (120 mL) was added to a mixture of tetrakis(triphenylphosphine)palladium (0) (0.87 g, 0.75 mmol) and 2,6-dichloropyrazine
(2.43 g, 16.3 mmol). After 15 min of stirring at room temperature, thiophene-3-boronic acid (2.09 g, 16.3 mmol) followed by aqueous Na₂CO₃ (2 M; 25 mL) were added. The mixture was heated at reflux for 2 h [TLC monitoring by SiO₂/*n-*hexane/ethyl acetate (85:15)]. The layers were separated and the light brownish water layer was extracted with ether (2 x 100 mL). The combined organic layers were dried (K₂CO₃), filtered and concentrated *in vacuo.* The brownish oil obtained was purified by silica gel chromatography (18 x 5 cm) eluting with *n*-hexane/ethyl acetate (85:15). This furnished 1.46 g (45%) of the title compound as an off-white solid. HRMS *m*/*z* calcd for C₈H₅ClN₂S (M)⁺ 195.9862, found 195.9868. Anal. (C₈H₅ClN₂S) C, H, N.

### Step 2: 2-(1-Piperazinyl)-6-(3-thienyl)pyrazine.

A mixture of the product from step 1 above (1.04 g, 5.29 mmol), piperazine (1.32 g, 15.3 mmol), and K₂CO₃ (0.81 g, 5.82 mmol) in acetonitrile (6 mL) was heated in a sealed pyrex flask at 85 °C for 8.5 h. The reaction mixture was diluted with dichloromethane, filtered, and concentated *in vacuo.* The semi-solid residue was purified by column chromatography on silica gel (18 x 5 cm) using CHCl₃/MeOH (9:1)
as eluent to furnish an oil. This material was redissolved in ethyl acetate, filtered, and concentrated *in vacuo.* This gave 0.98 g (75%) of the title compound as a yellow sticky oil. HRMS *m*/*z* calcd for C₁₂H₁₄N₄S (M)⁺ 246.0939, found 246.0943. Anal. (C₁₂H₁₄N₄S) C, H, N.

### EXAMPLE 17

### N-Benzyl-6-(1-piperazinyl)-2-pyrazinamine.

### Step 1: N-Benzyl-6-chloro-2-pyrazinamine.

A mixture of 2,6-dichloropyrazine (1.31 g, 8.8 mmol), benzylamine (1.15 g, 10.7 mmol) and K₂CO₃ (1.65 g, 11.9 mmol) in acetonitrile (6 mL) was heated at 85 °C for 13 h in a sealed pyrex flask. The reaction mixture was diluted with dichloromethane, filtered and concentrated *in vacuo.* The yellow solid residue was dissolved in a small volume of methanol and purified by silica gel chromatography (18 x 4 cm) using CHCl₃/MeOH (98:2) as eluent. A second purification (SiO₂; 16 x 4 cm) using CHCl₃ as eluent furnished 1.55 g (81%) of the title compound as a light yellow solid. HRMS *m*/*z* calcd for C₁₁H₁₀ClN₃ (M)⁺ 219.0563, found 219.0568. Anal. (C₁₁H₁₀ClN₃) C, H, N.

### Step 2: N-Benzyl-6-(1-piperazinyl)-2-pyrazinamine.

A mixture of the product from step 1 above (1.25 g, 5.7 mmol), piperazine (1.0 g, 11.6 mmol), and K₂CO₃ (1.0 g, 7.3 mmol) in dioxane (3 mL) was heated at 160 °C for 11h in a sealed pyrex flask. The reaction mixture was diluted with dichloromethane, filtered and concentrated *in vacuo.* The red-brownish residue was dissolved in a small volume of CHCl₃/MeOH (9:1) and purified by silica gel chromatography (15 x 4 cm) using CHCl₃/MeOH (95:5, followed by 9:1) as eluent. The free base was obtained as a brownish solid (0.9 g, 3.33 mol) that was redissolved in methanol (10 mL). Maleic acid (0.45 g, 3.83 mmol) in methanol (5 mL) was added and the salt was precipitated out by addition of ether. The salt was recrystallized from MeOH-ether and finally converted back to the free base by alkalinization (10% aqueous Na₂CO₃) and extraction with ether (5 x 60 mL). The combined ether layers were dried (K₂CO₃), filtered and concentrated. This furnished 0.36 g (23%) of the title compound as a light yellow powder. HRMS *m*/*z* calcd for C₁₅H₁₉N₅ (M)⁺ 269.1640, found 269.1641. Anal. (C₁₅H₁₉N₅) C, H, N.

### EXAMPLE 19

### 2-(2-Phenoxyethoxy)-6-(1-piperazinyl)pyrazine.

### Step 1: 2-Chloro-6-(2-phenoxyethoxy)pyrazine.

K-*t*-BuO (1.61 g, 14.3 mmol) was added portionwise to a stirred mixture of 2,6-dichloropyrazine (2.03 g, 13.6 mmol) and 2-phenoxyethanol (2.54 g, 18.4 mmol) in dioxane (8 mL) at 0 °C (ice-bath). After 5 min of stirring, the ice-bath was removed and the mixture was stirred at room temperature for 1h. The reaction mixture was diluted with ether, filtered and concentrated *in vacuo.* The oily residue was purified by silica gel chromatography (18 x 5cm) using *n*-hexane/ethyl acetate (92:8) as eluent.
This furnished 2.92 g (86%) of the title compound as a white solid. HRMS *m*/*z* calcd for C₁₂H₁₁ClN₂O₂ (M)⁺ 250.0509, found 250.0511. Anal. (C₁₂H₁₁ClN₂O₂) C, H, N.

### Step 2: 2-(2-Phenoxyethoxy)-6-(1-piperazinyl)pyrazine.

A mixture of the product from step 1 above (1.29 g, 5.15 mmol), piperazine (1.30 g, 15.1 mmol) and K₂CO₃ (0.71 g, 5.14 mmol) in acetonitrile (5 mL) was heated in a sealed pyrex flask at 100 °C for 4 h. The reaction mixture was diluted with dichloromethane, filtered, and concentrated *in vacuo.* The light-brown semi-solid residue was purified by silica gel chromatography (15 x 4 cm) using CHCl₃/MeOH (9:1) as eluent. Solvents were evaporated off and the residue was redissolved in ether/CHCl₃ (1:1). Filtration and concentration *in vacuo* furnished 1.05 g (68%) of the title compound as a white solid. HRMS *m*/*z* calcd for C₁₆H₂₀N₄O₂ (M)⁺ 300.1586, found 300.1578. Anal. (C₁₆H₂₀N₄O₂) C, H, N.

### EXAMPLE 20

### 2-(Benzyloxy)-6-(1-piperazinyl)pyrazine.*

A mixture of the product from example 13, step 2 (0.73 g, 3.68 mmol), benzyl alcohol (9.4 g, 87 mmol) and K-*t*-BuO was stirred at 125 °C for 4.5 h. The reaction mixture was purified by silica gel chromatography (13 x 5 cm) using CHCl₃/MeOH (7:3 followed by 9:1) as eluent. Solvents were evaporated off and the residue was redissolved in ethyl acetate. Filtration and concentration *in vacuo* furnished 0.90 g (90%) of the title compound as a beige oil. HRMS *m*/*z* calcd for C₁₅H₁₈N₄O (M)⁺ 270.1481, found 270.1482. Anal. (C₁₅H₁₈N₄O) C, H, N. *This compound was also characterized as its maleate salt: mp 155-156 °C. HRMS *m*/*z* calcd for C₁₅H₁₈N₄O (M)⁺ 270.1481, found 270.1482. Anal. (C₁₅H₁₈N₄O · C₄H₄O₄) C, H, N.

### EXAMPLE 21

### 2-Phenoxy-6-(1-piperazinyl)pyrazine.

A mixture of the product obtained in example 13, step 2 (1.97 g, 9.92 mmol), phenol (2.43 g, 25.8 mmol), CuO (1.0 g, 12.6 mmol), and K₂CO₃ (1.43 g, 10.3 mmol) in dioxane (2 mL) was stirred for 4.5 h at 165 °C in a sealed pyrex tube. The reaction mixture was diluted with CHCl₃ and filtered through a pad of Celite. The pad was washed with several portions of CHCl₃/MeOH (95:5). Solvent removal *in vacuo* furnished a dark brown oil which was purified by column chromatography on silica gel (14 x 5 cm) using CHCl₃/MeOH (95:5, followed by 90:10) as eluent. The brown oil (1.66 g) was subjected to repeated column chromatography, first on silica gel (18 x 4 cm) using CHCl₃/MeOH (9:1) as eluent and finally on alumina (4 x 5 cm) using ether/MeOH (95:5) as eluent. This furnished 1.37 g (54%) of the title compound as a light beige oil. HRMS *m*/*z* calcd for C₁₄H₁₆N₄O (M)⁺ 256.1324, found 256.1321. Anal. (C₁₄H₁₆N₄O) C, H, N.

### EXAMPLE 22

### 2-(1-Phenylethoxy)-6-(1-piperazinyl)pyrazine.

### Step 1: 2-Chloro-6-(1-phenylethoxy)pyrazine.

K-*t*-BuO (2,1 g, 18.7 mmol) was added to a stirred solution of 1-phenyl-1-ethanol (2.45 g, 20.1 mmol) in dioxane (30 mL) at 0 °C (ice-bath). After 10 min of stirring, 2,6-dichloropyrazine (2.49 g, 16.7 mmol) was added whereupon the reaction mixture turned orange colored. After being stirrred for a further 1.5 h, ether was added and the mixture was filtered. Concentration in vacuo furnished an orange colored oil that was purified by silica gel chromatography (15 x 5 cm) using *n*-hexane ethyl acetate (9:1) as eluent. This gave 3.29 g (84%) of the title compound as a colorless oil. HRMS *m*/*z* calcd for C₁₂H₁₁ClN₂O(M)⁺ 234.0560, found 234.0551.

### Step 2: 2-(1-Phenylethoxy)-6-(1-piperazinyl)pyrazine.

A mixture of the product from step 1 above (1.53 g, 6.5 mmol), piperazine (1.62 g, 18.9 mmol) and K₂CO₃ (0.90 g, 6.5 mmol) in acetonitrile (6 mL) was heated in a sealed pyrex flask at 90 °C for 3.5 h. The reaction mixture was diluted with dichloromethane, filtered and concentrated *in vacuo.* The semi-solid residue was purified by silica gel chromatography (13 x 4 cm) using CHCl₃/MeOH (9:1) as eluent. Solvents were evaporated off and the remaining oil was redissolved in CHCl₃, filtered through a short plug of alumina and concentrated *in vacuo.* This gave 1.34 g (72%) of the title compound as an oil that solidified when refrigerated. HRMS *m*/*z* calcd for C₁₆H₂₀N₄O (M)⁺ 284.1637, found 284.1650. Anal. (C₁₆H₂₀N₄O· C₄H₄O₄) C, H, N.

### EXAMPLE 23

### 2-(2-Fluoroethoxy)-6-(1-piperazinyl)pyrazine, Maleate.

### Step 1: 2-Chloro-6-(2-fluoroethoxy)pyrazine.

K-*t*-BuO (1.32 g, 11.8 mmol) was added portionwise to a stirred mixture of 2-fluoroethanol (2.16 g, 33.7 mmol) and 2,6-dichloropyrazine (1.61 g, 10.8 mmol) in dioxane (2 mL) at 0 °C (ice-bath). The reaction mixture was then stirred at room temperature for 1 hour, diluted with dichloromethane, filtered, and concentrated *in vacuo.* The residue was purified by column chromatography on silica gel (19 x 4 cm) using *n*-hexane/ethyl acetate (85:15) as eluent. This furnished 1.49 g (78%) of the title compound as a beige liquid. Anal. (C₆H₆FClN₂O) C, H, N.

### Step 2: 2-(2-Fluoroethoxy)-6-(1-piperazinyl)pyrazine, Maleate.

A mixture of the product from step 1 above (0.94 g, 5.31 mmol), piperazine (1.40 g, 16.3 mmol) and K₂CO₃ (0.81 g, 5.9 mmol) in acetonitrile (5 mL) was stirred at room temperature for 8.5 h and at 65 °C for 4 h. The reaction mixture was diluted with dichloromethane, filtered and concentrated *in vacuo.* The semi-solid residue was purified by silica gel chromatography (17 x 4 cm) using CHCl₃/MeOH (9:1) as eluent. Solvents were evaporated off and the remaining oil (0.73 g) was redissolved in ether/CHCl₃ (1:1) and filtered through a short (4 cm) plug of alumina using ether/MeOH (96:4) as eluent. Solvents were evaporated off and the residue was redissolved in ether and K₂CO₃ was added. Filtration and concentration *in vacuo* furnished 0.57 g (47%) of the free base of the title compound as an oil which was converted into its maleate salt. Recrystallization from MeOH/ether furnished 0.58 g of the title compound as a white powder. Anal. (C₁₀H₁₅FN₄O · C₄H₄O₄) C, H, N.

### EXAMPLE 24

### 2-(Cyclopentylmethoxy)-6-(1-piperazinyl)pyrazine.

### Step 1: 2-Chloro-6-(cyclopentylmethoxy)pyrazine.

K-*t*-BuO (1.65 g, 14.7 mmol) was added portionwise to a stirred mixture of cyclopentanemethanol (2.99 g, 29.9 mmol) and 2,6-dichloropyrazine (1.90 g, 12.8 mmol) in dioxane (6 mL) at 0 °C (ice-bath). The reaction mixture was then stirred for 2.5 h, while the temperature was allowed to reach room temperature. The reaction mixture was diluted with dichloromethane/ether (1:1), filtered, and concentated *in vacuo.* The beige liquid was purified by column chromatography on silica gel (18 x 4 cm) using *n*-hexane/ethyl acetate (94:6) as eluent. Two chromatographic runs afforded 1.66 g (61%) of the title compound as a colorless oil. HRMS *m*/*z* calcd for C₁₀H₁₃ClN₂O (M)⁺ 212.0716, found 212.0723. Anal. (C₁₀H₁₃ClN₂O) C, H, N.

### Step 2: 2-(Cyclopentylmethoxy)-6-(1-piperazinyl)pyrazine.

A mixture of the product from step 1 above (1.12 g, 5.27 mmol), piperazine (1.36 g, 15.8 mmol) and K₂CO₃ (0.77 g, 5.6 mmol) in acetonitrile (5 mL) was stirred at 100 °C for 4.5 h in a sealed pyrex flask. The reaction mixture was diluted with dichloromethane, filtered and concentrated *in vacuo.* The semi-solid residue was purified by silica gel chromatography (15 x 4 cm) using CHCl₃/MeOH (9: 1) as eluent. Solvents were evaporated off and the remaining thick oil was redissolved in ether. Filtration and concentration *in vacuo* furnished 1.02 g (74%) of the title compound as a beige oil. HRMS *m*/*z* calcd for C₁₄H₂₂N₄O (M)⁺ 262.1794, found 262.1800. Anal. (C₁₄H₂₂N₄O) C, H, N.

### EXAMPLE 25

### 2-Benzyl-6-(1-piperazinyl)pyrazine.

### Step 1: 2-Chloro-6-benzylpyrazine

The title compound was prepared in a 20 mmol-scale according to the procedure described in WO 94/26715 with a slight modification. The reaction was carried out at 50 °C for 8 h followed by 10 h at room temperature. Yield: 0.75 g (18%). HRMS *m*/*z* calcd for C₁₁H₉ClN₂ (M)⁺ 204.0454, found 204.0450.

### Step 2: 2-Benzyl-6-(1-piperazinyl)pyrazine.

A mixture of the product from step 1 above (0.83 g, 4.0 mmol), piperazine (1.1 g, 12.8 mmol) and K₂CO₃ (0.62 g, 4.49 mmol) in acetonitrile (7 mL) was stirred at 85 °C for 8.5 h. Thr reaction mixture was diluted with dichloromethane, filtered and concentrated *in vacuo.* The semi-solid residue was purified by silica gel chromatography (20 x 4 cm) using CHCl₃/MeOH (9: 1) as eluent. The resulting oil was redissolved in CHCl₃ and filtered through a short (4 cm) plug of alumina using ether/MeOH (96:4) as eluent. Solvent removal *in vacuo* furnished 0.59 g (57%) of the title compound as an oil which became semi-solid upon refrigeration. HRMS *m*/*z* calcd for C₁₅H₁₈N₄ (M)⁺ 254.1531, found 254.1527. Anal. (C₁₅H₁₈N₄) C, H, N.

### EXAMPLE 26

### 2-(3,4-Dihydro-2H-chromen-4-yloxy)-6-(1-piperazinyl)pyrazine, Maleate.

### Step 1: 2-Chloro-6-(3,4-dihydro-2H-chromen-4-yloxy)pyrazine.

K-*t*-BuO (1.28 g, 11.42 mmol) was added to a stirred solution of 4-chromanol (1.81 g, 12.0 mmol) in dioxane (30 mL) at 0 °C (ice-bath). After being stirred for 5 min at room temperature, the mixture was chilled to 0 °C (ice-bath) and 2,6-dichloropyrazine (1.49 g, 10.0 mmol) was added. The reaction mixture was stirred at room temperature for 15 min and diluted with dichloromethane. Filtration and concentration *in vacuo* furnished an orange thick oil which was purified by chromatography on silica gel (15 x 4 cm) using *n*-hexane/ethyl acetate (8:2) as eluent. This furnished 1.87 g (71%) of the title compound as a colorless oil. HRMS *m*/*z* calcd for C₁₃H₁₁ClN₂O₂ (M)⁺ 262.0509, found 262.0520. Anal. (C₁₃H₁₁ClN₂O₂) C, H, N.

### Step 2: 2-(3,4-Dihydro-2H-chromen-4-yloxy)-6-(1-piperazinyl)pyrazine, Maleate.

A mixture of the product from step 1 above (1.53 g, 5.81 mmol), piperazine (1.45 g, 16.9 mmol) and K₂CO₃ (0.80 g, 5.81 mmol) in acetonitrile (10 mL) was heated in a sealed pyrex flask at 110°C for 6.5 h. The reaction mixture was diluted with CHCl₃, filtered and concentrated *in vacuo.* The semi-solid residue was purified by column chromatography on silica (13 x 4 cm) using CHCl₃/MeOH (9: 1) as eluent. The free base of the title compound was obtained as a viscous oil (1.76 g, 97%) which was converted to its maleate salt. Recrystallization from MeOH/ether furnished 1.78 g (74%) of the title compound as a light yellow powder: mp 179.5-182 °C. HRMS *m*/*z* calcd for C₁₇H₂₀N₄O₂ (M)⁺ 312.1586, found 312.1581. Anal. (C₁₇H₂₀N₄O₂· C₄H₄O₄) C, H, N.

### EXAMPLE 27

### 2-[2-(4-Dimethylaminophenyl)ethoxy]-6-(1-piperazinyl)pyrazine, Maleate.

### Step 1: 2-Chloro-6-[2-(4-dimethylaminophenyl)ethoxy]pyrazine.

K-*t*-BuO (2.27 g, 20.3 mmol) was added to a stirred solution of 4-(dimethylamino)phenethyl alcohol (3.55 g, 21.5 mmol) in dioxane (35 mL) at 0 °C (ice-bath). After being stirred for 5 min at 0 °C and 12 min at room temperature, the mixture was chilled to 0 °C (ice-bath) and 2,6-dichloropyrazine (2.62 g, 17.6 mmol) was added. The reaction mixture was stirred at 0 °C for 5 min and at room temperature for 20 min and diluted with dichloromethane/ether (1:1). Filtration through a pad of Celite, covered with K₂CO₃, and concentration *in vacuo* furnished a yellow oil. This material was purified by chromatography on silica gel (15 x 5 cm) using *n*-hexane/ethyl acetate (85:15). A second chromatograhic run on silica gel (14 x 5 cm) using *n*-hexane/ethyl acetate (88:12) furnished 3.91 g (80%) of the title compound as a colorless oil. Anal. (C₁₄H₁₆ClN₃O) C, H, N.

### Step 2: 2-[2-(4-Dimethylaminophenyl)ethoxy]-6-(1-piperazinyl)pyrazine, Maleate.

A mixture of the product from step 1 above (1.83 g, 6.59 mmol), piperazine (1.69 g, 19,6 mmol) and K₂CO₃ (0.92 g, 6.7 mmol) in acetonitrile (25 mL) was heated under reflux for 8.5 h. The reaction mixture was diluted with dichloromethane, filtered through a pad of Celite, and concentrated *in vacuo.* The semi-solid residue was purified by column chromatography on silica (13 x 4 cm) using CHCl₃/MeOH (92:8) as eluent. The free base of the title compound was obtained as a beige viscous oil (1.17 g, 54%) which was converted into its maleate salt. Recrystallization from MeOH/ether furnished 1.33 g of the title compound as a light yellow powder. HRMS *m*/*z* calcd for C₁₈H₂₅N₅O (M)⁺ 327.2059, found 327.2066. Anal. (C₁₈H₂₅N₅O - C₄H₄O₄) C, H, N.

### EXAMPLE 28

### 2-[2-(1H-Indol-1-yl)ethoxy]-6-(1-piperazinyl)pyrazine, Maleate.

### Step 1: 2-(1H-Indol-1-yl)ethanol.*

A mixture of indole (5.71 g, 48.7 mmol), ethylene carbonate (4.72 g, 53.6 mmol) and K₂CO₃ (6.73 g, 48.7 mmol) in DMF (20 mL) was heated under reflux for 2 h. The reaction mixture was diluted with dichloromethane, filtered, and concentrated *in vacuo.*
The light brown oily residue was purified by chromatography on silica gel (13 x 6 cm)
using *n*-hexane/ethyl acetate (1:1) as eluent. This gave 1.78 g (23%) of the title compound as a beige oil. HRMS *m*/*z* calcd for C₁₀H₁₁NO (M)⁺ 161.0841, found 161.0849. Anal. (C₁₀H₁₁NO · 0.1H₂O) C, H, N. *Previously described in a) J. Med. Chem. 1992, 35, 994-1001; b) ibid. 1998, 41, 1619-1630.

### Step 2: 2-Chloro-6-[2-(1H-indol-1-yl)ethoxy] pyrazine.

K-*t*-BuO (0.67 g, 5.93 mmol) was added to a stirred solution of the product obtained in step 1 above (0.67 g, 5.93 mmol) in dioxane (20 mL) at 0 °C (ice-bath). After being stirred for 7 min at 0 °C and 5 min at room temperature, the mixture was chilled to 0 °C (ice-bath) and 2,6-dichloropyrazine (2.62 g, 17.6 mmol) was added. The yellowish reaction mixture was stirred at 0 °C for 20 min and at room temperature for 10 min and then diluted with dichloromethane. Drying (K₂CO₃), filtration, and concentration *in vacuo* furnished a beige oil. This material was purified by chromatography on silica gel (13 x 4 cm) using *n*-hexane/ethyl acetate (80:20). This furnished 1.39 g (94%) of the title compound as an oil that solidified upon standing. HRMS *m*/*z* calcd for C₁₄H₁₂ClN₃O (M)⁺ 273.0669, found 273.0671.
Anal. (C₁₄H₁₂ClN₃O) C, H, N.

### Step 3: 2-[2-(1H-Indol-1-yl)ethoxy]-6-(1-piperazinyl)pyrazine, Maleate.

A mixture of the product from step 1 above (1.05 g, 3.84 mmol), piperazine (0.96 g, 11.1 mmol) and K₂CO₃ (0.53 g, 3.84 mmol) was heated at 85 °C for 7 h. The reaction mixture was diluted with CHCl₃, filtered, and concentrated *in vacuo.* The semi-solid residue was purified by chromatography on silica gel (11 x 4 cm) using CHCl₃/MeOH (9:1) as eluent. The resulting oil was redissolved in CHCl₃ and filtered through a short (4 cm) plug of alumina covered by K₂CO₃ using CHCl₃ as eluent. Solvent removal *in vacuo* furnished 1.02 g (82%) of the free base of the title compound as a beige oil which was converted into its maleate. Recrystallization from MeOH/ether furnished 1.00 g (75%) of the title compound as a light yellow powder: mp 160.5-163 °C. HRMS *m*/*z* calcd for C₁₈H₂₁N₅O (M)⁺ 323.1746, found 323.1757.
Anal. (C₁₈H₂₁N₅O · C₄H₄O₄) C, H, N.

### EXAMPLE 29

### 2-[2-(1H-Indol-3-yl)ethoxy]-6-(1-piperazinyl)pyrazine.

### Step 1: 2-Chloro-6-[2-(1H-indol-3-yl)ethoxy]pyrazine.

K-*t-*BuO (2.32 g, 20.6 mmol) was added to a stirred solution of tryptophol (1.7 g, 10.6 mmol) in dioxane (30 mL) at 0 °C (ice-bath). After being stirred for 10 min at 0 °C and 10 min at room temperature, the mixture was chilled to 0 °C (ice-bath) and 2,6-dichloropyrazine (1.37 g, 9.17 mmol) was added. The yellowish reaction mixture was stirred at 0 °C for 30 min and for a further 20 min at room temperature. The mixture was diluted with dichloromethane, filtered, and concentrated *in vacuo* to furnish a brownish oil. This material was purified by chromatography on silica gel (14 x 5 cm) using *n-*hexane/ethyl acetate (75:25). This furnished 1.38 g (55%) of the title compound as a beige solid. Purity >90% by ¹H NMR in CDCl₃.

### Step 2: 2-[2-(1H-Indol-3-yl)ethoxy]-6-(1-piperazinyl)pyrazine.

A mixture of the product from Step 1 above (1.07 g, 3.90 mmol), piperazine (0.98 g, 11.3 mmol) and K₂CO₃ (0.54 g, 3.9 mmol) in acetonitrile (11 mL) was heated at 85 °C for 5 h and at 110 °C for 8 h in a sealed pyrex flask. The reaction mixture was diluted with CHCl₃, filtered, and concentrated *in vacuo.* The semi-solid residue was purified by chromatography on silica gel (11 x 4 cm) using CHCl₃/MeOH (9:1) as eluent. The resulting oil was redissolved in CHCl₃ and filtered through a short plug of alumina, covered by K₂CO₃, using CHCl₃ as eluent. Solvent removal *in vacuo* furnished 0.50 g (23%) of the title compound as an oil that solidified upon standing: mp 133-135 °C. HRMS *m*/*z* calcd for C₁₈H₂₁N₅O (M)⁺ 323.1746, found 323.1763.
Anal. (C₁₈H₂₁N₅O)
C, H, N.

### EXAMPLE 39

### (2S)-1-[6-(Benzyloxy)-2-pyrazinyl]-2-methylpiperazine.

### Step 1: (3S)-3-Methyl-1-tritylpiperazine.

To a solution of 2-(*S*)-methylpiperazine (2.62 g, 26.2 mmol) in dichloromethane (100 mL) was trityl chloride (7.30 g, 26.2 mmol) added and the mixture was stirred at ambient temperature for 1.5 h. The organic phase was washed (x 1) with 1 M aqueous K₂CO₃, water, and brine. Drying (MgSO₄) and solvent removal *in vacuo* furnished a quantitative yield of the title compound as a glassy oil which solidified upon standing. This material was used directly in the next step.

### Step 2: (2S)-1-(6-Chloro-2-pyrazinyl)-2-methylpiperazine.

A mixture of 2-6-dichloropyrazine (1.10 g, 7.39 mmol), and the product from step 1 above (2.30 g, 6.72 mmol) and K₂CO₃ (1.0 g, 7.39 mmol) in dry DMF (40 mL) was stirred at 110 °C over night. The dark reaction mixture was filtered through a plug of silica and the solvent was removed under reduced pressure. The remaining oil was dissolved in CHCl₃/*n-*heptane (1:1) and filtered through a second plug of silica. The solvent was evaporated and the remaining yellow oil was suspended in EtOH (80 mL).
4 M aqueous HCl (2 mL) was added and the mixture was ultrasonicated for 20 min. The solvent was evaporated and the remaining oil was taken up between water/CHCl₃. The organic phase was made alkaline (11 M aqueous NaOH) and extracted twice with CHCl₃. The combined dried (MgSO₄) organic layers were concentrated *in vacuo* to afford 0.75 g (54%) of the title compound as a yellow oil.
MS *m*/*z* 212/214 (M)⁺ (³⁵Cl/ ³⁷Cl-isotope pattern). HRMS *m*/*z* calcd for C₉H₁₃ClN₄ (M)⁺ 212.0829, found 212.0827.

### Step 3: (2S)-1-[6-(Benzyloxy)-2-pyrazinyl]-2-methylpiperazine, Acetate.

To a solution of (2*S*)-1-(6-chloro-2-pyrazinyl)-2-methylpiperazine (prepared in step 2 above; 0.16 g, 0.72 mmol) and benzyl alcohol (0.12 g, 1.1 mmol) in DMF (4 mL) was Na-*t-*BuO (0.14 g, 1.4 mmol) added and the mixture was stirred at 150 °C overnight. The solvent was evaporated off under reduced pressure and the residue taken up between CHCl₃/H₂O. The organic phase was concentrated and the crude product was purified by preparative C18-HPLC using acetonitrile/H₂O/HOAc with UV detection at 254 nm. Yield: 1 mg (0.4%). MS *m*/*z* 284 (M)⁺. HRMS *m*/*z* calcd for C₁₆H₂₀N₄O (M)⁺ 284.1637, found 284.1640.

### EXAMPLE 41

### 1-[6-(Benzyloxy)-2-pyrazinyl]-2-ethylpiperazine, Acetate.

### Step 1: 1-Benzyl-3-ethylpiperazine.*

Benzyl bromide (38.7 g, 0.22 mol) was added in portions to a chilled (~0 °C) solution of 2-ethylpiperazine (25 g, 0.22 mol) in DMF (150 mL) with such a rate that the temperature did not exceed 20 °C. The mixture was stirred for 1 h, the solvent was evaporated and the residue was partitioned between CHCl₃/0.5 M aqueous HCl. The aqueous phase was made alkaline (11 M NaOH) and extracted three times with CHCl₃. The combined organic phases were dried (MgSO₄) and concentrated. The resulting oil was purified by column chromatography on silica using CHCl₃, followed by CHCl₃/MeOH/NH₄OH (95:5:0.3) as eluents to give 31.6 g (70%) of the title compound as a yellowish oil. Anal. (C₁₃H₂₀N₂) H, N; C: calcd, 76.42; found, 75.85. *Described in WO 00/76984.

### Step 2: 4-Benzyl-1-(6-chloro-2-pyrazinyl)-2-ethylpiperazine.

The title compound was prepared according to the procedure of example 39, step 2, starting from the product obtained in step 1 above (4.60 g, 22.5 mmol), 2,6-dichloropyrazine (3.90 g, 26.2 mmol) and K₂CO₃ (6.22 g, 45.0 mmol). Yield: 6.15 g (86%). MS *m*/*z* 316/318 (M)⁺ (³⁵Cl/ ³⁷Cl-isotope pattern). HRMS *m*/*z* calcd for C₁₇H₂₁ClN₄ (M)⁺ 316.1455, found 316.1455.

### Step 3: 1-(6-Chloro-2-pyrazinyl)-2-ethylpiperazine.

1-Choroethyl chloroformate (4.16 g, 29.1 mmol) was added dropwise under 2 h to a stirred solution of the product obtained in step 2 above (6.15 g, 19.4 mmol) in dry dichloromethane (75 mL) at 0 °C. After being stirred at room temperature for 15 h, the reaction mixture was concentrated *in vacuo* and methanol was added. The mixture was heated at reflux for 2 h and concentrated. The residue was dissolved in CHCl₃ and passed through a short (4 cm) plug of silica gel using CHCl₃/MeOH (8:2) as eluent. Solvents were evaporated off and the residue was purified by chromatography on silica gel (12 x 5 cm) using CHCl₃/MeOH/Et₃N (95:5:0.2) as eluent. This provided 1.9 g (43%) of the title compound as an oil. MS *m*/*z* 226/228 (M)⁺ (³⁵Cl/ ³⁷Cl-isotope pattern). HRMS *m*/*z* calcd for C₁₀H₁₅ClN₄ (M)⁺ 226.0985, found 226.0986.

### Step 4: 1-[6-(Benzyloxy)-2-pyrazinyl]-2-ethylpiperazine, Acetate.

The title compund was prepared according to the procedure of example 39, step 3, starting from the product of step 3 above (0.163 g, 0.72 mmol), benzyl alcohol (0.12 g, 1.08 mmol) and Na-*t-*BuO (0.14 g, 1.4 mmol). Purity 90% (HPLC). MS *m*/*z* 298 (M)⁺. HRMS *m*/*z* calcd for C₁₇H₂₂N₄O (M)⁺ 298.1794, found 298.1802.

### EXAMPLE 42

### 2-[(4-Fluorobenzyl)oxy]-6-(1-piperazinyl)pyrazine.

4-Fluorobenzyl alcohol (0.189 g, 1.50 mmol) was dissolved in THF (1 mL) and treated with NaH (0.065 g, 55% dispersion in mineral oil, 1.5 mmol). The reaction mixture was stirred at room temperature for 3 h. A solution of 2,6-dichloropyrazine (1.57 g, 10.5 mmol) in THF (7 mL) was added and the resulting mixture was stirred for 4 h at room temperature. Piperazine (0.580 g, 6.75 mmol) and K₂CO₃ (0.43 g, 4.5 mmol) were added and the mixture was stired at 60 °C over night. Filtration, concentration, and purification by chromatography on silica gel using ethyl acetate/acetic acid/methanol/water (24:3:3:2) as eluent furnished 0.20 g (46%) of the title compound as a white solid: mp 183 °C. HRMS *m*/*z* calcd for C₁₅H₁₇FN₄O (M)⁺ 288.1386, found 288.1380. Anal. (C₁₅H)₇FN₄O 2.6H₂O) C, H, N.

### EXAMPLE 43

### 2-[(4-Methoxybenzyl)oxy]-6-(1-piperazinyl)pyrazine, Acetate.

The title compound was prepared according to the procedure of example 42 starting from 4-methoxybenzyl alcohol (0.207 g, 1.50 mmol) and was isolated as a yellow solid. Yield: 0.79 g (67%). HRMS *m*/*z* calcd for C₁₆H₂₀N₄O₂ (M)⁺ 300.1586, found 300.1584.

### EXAMPLE 44

### 2-[2-(4-Fluorophenyl)ethoxy]-6-(1-piperazinyl)pyrazine, 0.5 Acetate.

The title compound was prepared according to the procedure of example 42 starting from 2-(4-fluorophenyl)ethanol (0.210 g, 1.50 mmol) and was isolated as a yellow solid. Yield: 0.145 g (27%). HRMS *m*/*z* calcd for C₁₆H₁₉FN₄O (M)⁺ 302.1543, found 302.1554. Anal. (C₁₆H₁₉FN₄O · 0.5CH₃COOH · H₂O) C, H, N.

### EXAMPLE 50

### 2-[1-(2-Methoxyphenyl)ethoxy]-6-(1-piperazinyl)pyrazine.

### Step 1: 2-Chloro-6-[1-(2-methoxyphenyl)ethoxy]pyrazine.

K-*t-*BuO (0.67 g, 5.97 mmol) was added to a stirred solution of 1-(2-methoxyphenyl)ethanol (0.96 g, 6.28 mmol) in dioxane (15 mL) at 0 °C (ice-bath). After 5 min of stirring at room temperature, the reaction mixture was chilled to 0 °C (ice-bath) and 2,6-dichloropyrazine (0.78 g, 5.23 mmol) was added whereupon the reaction mixture turned yellow. After being stirrred for 35 min, dichloromethane and K₂CO₃ were added and the mixture was filtered. Concentration *in vacuo* furnished a yellow oil that was purified by silica gel chromatography (15 x 4 cm) using *n-*hexane ethyl acetate (8:2) as eluent. This gave 1.21 g (92%) of the title compound as a colorless oil. HRMS *m*/*z* calcd for C₁₃H₁₃ClN₂O₂ (M)⁺ 264.0666, found 264.0677.
Anal. (C₁₃H₁₃ClN₂O₂) C, H, N.

### Step 2: 2-[1-(2-Methoxyphenyl)ethoxy]-6-(1-piperazinyl)pyrazine.

A mixture of the product from step 1 above (0.93 g, 3.53 mmol) piperazine (0.88 g, 10.2 mmol) and K₂CO₃ (0.49 g, 3.53 mmol) in acetonitrile (7 mL) was heated in a sealed pyrex flask at 90 °C for 6.5 h. The reaction mixture was diluted with dichloromethane, filtered and concentrated *in vacuo.* The semi-solid residue was purified by silica gel chromatography (13 x 4 cm) using CHCl₃/MeOH (9:1) as eluent. Solvents were evaporated off and the remaining oil was redissolved in CHCl₃, filtered through a short plug of alumina, covered with K₂CO₃, using CHCl₃ as eluent. Solvent removal *in vacuo* gave 0.74 g (67%) of the title compound as a beige oil. HRMS *m*/*z* calcd for C₁₇H₂₂N₄O₂ (M)⁺ 314.1743, found 314.1733. Anal. (C₁₇H₂₂N₄O₂ · 0.5H₂O) C, H, N.

### EXAMPLE 52

### 2-[2-(2,3-Dimethoxyphenyl)ethoxy]-6-(1-piperazinyl)pyrazine, Maleate.

The title compound was prepared according to the procedure of example 50, step 2, starting from 2-chloro-6-[2-(2,3-dimethoxyphenyl)ethoxy]pyrazine [0.65 g, 2.2 mmol; obtained according to the procedure of example 50, step 1, starting from 2-(2,3-dimethoxyphenyl)ethan-1-ol], piperazine (0.57 g, 6.7 mmol) and K₂CO₃ (0.31 g, 2.22 mmol). The free base of the title compound was converted into its maleate salt. Recrystallization from MeOH-ether furnished 0.45 g (44%) of the title compound. Purity 98% (HPLC). MS *m*/*z* 345 (M + H)⁺. HRMS *m*/*z* calcd for C₁₈H₂₄N₄O₃ (M)⁺ 344.1848, found 344.1861.

### EXAMPLE 53

### 2-[2-(2-Fluorophenyl)ethoxy]-6-(1-piperazinyl)pyrazine, Maleate.

The title compound was prepared according to the procedure of example 50, step 2, starting from 2-chloro-6-[2-(2-fluorophenyl)ethoxy]pyrazine (2.76 g, 10.9 mmol; obtained according to the procedure of example 50, step 1, starting from 2-fluorophenethyl alcohol), piperazine (2.91 g, 33.8 mmol) and K₂CO₃ (1.51 g, 10.9 mmol). The yield of the free base of the title compound was 1.88 g (57%) which was converted into its maleate salt. Recrystallization from MeOH-ether furnished 2.11 g of the title compound. Purity 100% (HPLC). MS *m*/*z* 303 (M + H)⁺. HRMS *m*/*z* calcd for C₁₆H₁₉FN₄O (M)⁺ 302.1543, found 302.1550.

### EXAMPLE 54

### 2-[(2,3-Dimethoxybenzyl)oxy]-6-(1-piperazinyl)pyrazine.

The title compound was prepared according to the procedure of example 50, step 2, starting from 2-chloro-6-[(2,3-dimethoxybenzyl)oxy]pyrazine (2.51 g, 8.93 mmol; obtained according to the procedure of example 50, step 1, starting from 2,3-dimethoxybenzyl alcohol), piperazine (2.38 g, 27.7 mmol) and K₂CO₃ (1.23 g, 8.9 mmol). The yield of the title compound was 1.66 g (56%) which was obtained as an oil. Purity 100% (HPLC). MS *m*/*z* 331 (M + H)⁺. HRMS *m*/*z* calcd for C₁₇H₂₂N₄O₃ (M)⁺ 330.1692, found 330.1690.

### EXAMPLE 55

### 2-(2,3-Dihydro-1H-inden-1-ylmethoxy)-6-(1-piperazinyl)pyrazine, Maleate.

The title compound was prepared according to the procedure of example 50, step 2, starting from 2-chloro-6-(2,3-dihydro-1*H-*inden-1-ylmethoxy)pyrazine (3.22 g, 13.1 mmol; obtained according to the procedure of example 50, step 1, starting from 1-indanol), piperazine (3.49 g, 40.5 mmol) and K₂CO₃ (1.8 g, 13.0 mmol). The yield of the free base of the title compound was 2.19 g (57%) which was obtained as an oil. The free base was converted into its maleate salt. Purity 100% (HPLC). MS *m*/*z* 296 (M)⁺. HRMS *m*/*z* calcd for C₁₇H₂₀N₄O (M)⁺ 296.1637, found 296.1643.

### EXAMPLE 56

### 2-(4-Phenoxybutoxy)-6-(1-piperazinyl)pyrazine.

The title compound was prepared according to the procedure of example 50, step 2, starting from 2-chloro-6-(4-phenoxybutoxy)pyrazine (1.99 g, 7.14 mmol; obtained according to the procedure of example 50, step 1, starting from 4-phenoxy-1-butanol*), piperazine (1.84 g, 21.4 mmol) and K₂CO₃ (0.99 g, 7.14 mmol). The yield of the title compound was 1.52 g (65%) which was obtained as an oil. Purity 100% (HPLC). MS *m*/*z* 329 (M + H)⁺. HRMS *m*/*z* calcd for C₁₈H₂₄N₄O₂ (M)⁺ 328.1899, found 328.1894. *Prepared by reduction (LiAlH₄) of the corresponding acid (*cf*. J. Org. Chem. 1965, 30, 2441-2447; ibid. 1968, 33, 2271-2284).

### EXAMPLE 57

### 2-[(5-Phenoxypentyl)oxy]-6-(1-piperazinyl)pyrazine.

The title compound was prepared according to the procedure of example 50, step 2, starting from 2-chloro-6-[(5-phenoxypentyl)oxy]pyrazine (2.06 g, 7.03 mmol; obtained according to the procedure of example 50, step 1, starting from 5-phenoxy-1-pentanol*), piperazine (1.88 g, 21.8 mmol) and K₂CO₃ (0.97 g, 7.03 mmol). The yield of the title compound was 1.15 g (48%) which was obtained as a white solid. Purity 100% (HPLC). MS *m*/*z* 343 (M + H)⁺. HRMS *m*/*z* calcd for C₁₉H₂₆N₄O₂ (M)⁺ 342.2056, found 342.2054. *Described in. J. Org. Chem. 1968, 33, 2271-2284.

### EXAMPLE 58

### 2-[(2,5-Dimethoxybenzyl)oxy]-6-(1-piperazinyl)pyrazine.

The title compound was prepared according to the procedure of example 50, step 2, starting from 2-chloro-6-[(2,5-dimethoxybenzyl)oxy]pyrazine (1.02 g, 3.63 mmol; obtained according to the procedure of example 50, step 1, starting from 2,5-dimethoxybenzyl alcohol), piperazine (0.94 g, 10.9 mmol) and K₂CO₃ (0.50 g, 3.63 mmol). The yield of the title compound was 0.64 g (53%) which was obtained as a beige solid. Purity 100% (HPLC). MS *m*/*z* 331 (M + H)⁺. HRMS *m*/*z* calcd for C₁₇H₂₂N₄O₃ (M)⁺ 330.1692, found 330.1692.

### EXAMPLE 59

### 2-[2-(3,4-Dimethoxyphenyl)ethoxy]-6-(1-piperazinyl)pyrazine, Maleate.

The title compound was prepared according to the procedure of example 50, step 2, starting from 2-chloro-6-[2-(3,4-dimethoxyphenyl)ethoxy]pyrazine [2.13 g, 7.23 mmol; obtained according to the procedure of example 50, step 1, starting from 2-(3,4-dimethoxyphenyl)ethan-1-ol], piperazine (1.93 g, 22.4 mmol) and K₂CO₃ (1.0 g, 7.2 mmol). The yield of the title compound was 1.72 g (69%) which was obtained as a beige oil. Purity 100% (HPLC). MS *m*/*z* 345 (M + H)⁺. The free base was converted into ints maleate salt. HRMS *m*/*z* calcd for C₁₈H₂₄N₄O₃ (M)⁺ 344.1848, found 344.1832.

### EXAMPLE 60

### 2-{[2-(2-Phenylethyl)benzyl]oxy}-6-(1-piperazinyl)pyrazine.

The title compound was prepared according to the procedure of example 50, step 2, starting from 2-chloro-6-{[2-(2-phenylethyl)benzyl]oxy}pyrazine (1.72 g, 5.30 mmol); obtained according to the procedure of example 50, step 1, starting from 2-phenethylbenzyl alcohol), piperazine (1.37 g, 16.0 mmol) and K₂CO₃ (0.73 g, 5.3 mmol). The yield of the title compound was 1.38 g (69%) which was obtained as an oil. Purity 100% (HPLC). MS *m*/*z* 375 (M + H)⁺. HRMS *m*/*z* calcd for C₂₃H₂₆N₄O (M)⁺ 374.2107, found 374.2113.

### EXAMPLE 61

### 2-[(3-Phenoxybenzyl)oxy]-6-(1-piperazinyl)pyrazine.

The title compound was prepared according to the procedure of example 50, step 2, starting from 2-chloro-6-[(3-phenoxybenzyl)oxy]pyrazine (1.99 g, 6.36 mmol; obtained according to the procedure of example 50, step 1, starting from 3-phenoxybenzyl alcohol), piperazine (1.94 g, 22.5 mmol) and K₂CO₃ (0.88 g, 6.4 mmol). The yield of the title compound was 1.58 g (69%) which was obtained as an oil. Purity 100% (HPLC). MS *m*/*z* 363 (M + H)⁺. HRMS *m*/*z* calcd for C₂₁H₂₂N₄O₂ (M)⁺ 362.1743, found 362.1739.

### EXAMPLE 62

### (2R)-1-[6-(Benzyloxy)-2-pyrazinyl]-2-methylpiperazine, Maleate.

### Step 1: (3R)-3-Methyl-1-tritylpiperazine.

The title compound was prepared according to the procedure of Example 39, step 1, with the exception that (2*R*)-methylpiperazine was substituted for (2*S*)-methylpiperazine. The title compound was obtained as a light yellow crispy solid.

### Step 2: (2R)-1-(6-Chloro-2-pyrazinyl)-2-methylpiperazine, Maleate.

A mixture of 2.6-dichloropyrazine (2.33 g, 15.7 mmol), the product from step I above (5.11 g, 14.9 mmol) and K₂CO₃ (3.09 g, 22.4 mmol) in dry DMF (50 mL) was stirred at 120 °C for 7.5 h. The dark reaction mixture was diluted with ether and solids were filtered off. The filter cake was washed with CHCl₃. The filtrate was concentrated under reduced pressure. The residue was redissolved in CHCl₃ (150 mL) and 5M aqueous HCl (20 mL) was added and the mixture was stirred at room temperature 8.5 h. A solution of 5 M aqueous NaOH (25 mL) was added carefully and the layers were separated. The aqueous layer was extracted with CHCl₃ (2 x 150 mL). The combined, dried (K₂CO₃), organic phases were concentrated *in vacuo.* The brownish oily residue was purified by silica gel chromatography (bed size: I I x 6 cm) using CHCl₃/MeOH (92:8) as eluent. The yield of the free base of the title compound was 1.74 g (55%) which was obtained as a tan colored oil. A portion (0.41 g, 1.9 mmol) of the free base was converted into its maleate salt. Purity 99% (HPLC). HRMS *m*/*z* calcd for C₉H₁₃ClN₄ (M)⁺ 212.0829, found 212.0819.

### Step 3: (2R)-1-[6-(Benzyloxy)-2-pyrazinyl]-2-methylpiperazine, Maleate.

K-*t-*BuO (2.07 g, 18.4 mmol) was added to a mixture of (2*R*)-1-(6-chloro-2-pyrazinyl)-2-methylpiperazine (prepared in step 2 above; 1.31 g, 6.15 mmol) and benzyl alcohol (10.0 g, 92.5 mmol). After being stirred for 7 h at 95 °C, the mixture was applied onto a bed of silica (12 x 6 cm). Elution with CHCl₃/MeOH (97:3 followed by 92:8) furnished 1.44 g (82%) of the free base of the title compound as a light yellow oil. The free base was converted into its maleate salt. Purity 99% (HPLC). MS *m*/*z* 284 (M)⁺. HRMS *m*/*z* calcd for C₁₆H₂₀N₄O (M)⁺ 284.1637, found 284.1633.

### EXAMPLE 65

### 2-(1-Piperazinyl)-6-{[3-(1H-pyrrol-1-yl)-2-thienyl]methoxy}pyrazine.

### Step 1: 2-Chloro-6-{[3-(1H-pyrrol-1-yl)-2-thienyl]methoxy}pyrazine.

The title compound was prepared according to the procedure of example 50, step 1, starting from 3-(pyrrol-1-yl)thiophene-2-methanol (2.5 g, 14 mmol), K-*t-*BuO (1.43 g, 12.7 mmol) and 2,6-dichloropyrazine (1.73 g, 11.6 mmol). The yield of the title compound was 3.05 g (90%) and was obtained as an oil. Anal. (C₁₃H₁₀ClN₃OS) C, H, N.

### Step 2: 2-(1-Piperazinyl)-6-{[3-(1H-pyrrol-1-yl)-2-thienyl]methoxy}pyrazine.

The title compound was prepared according to the procedure of example 50, step 2, starting from the product obtained in step 1 above (1.78 g, 6.10 mmol), piperazine (1.58 g, 18.3 mmol) and K₂CO₃ (0.86 g, 6.2 mmol). The yield of the title compound was 1.43 g (69%) and was obtained as a beige solid. HRMS *m*/*z* calcd for C₁₇H₁₉N₅OS (M)⁺ 341.1310, found 341.1301.

### EXAMPLE 66

### 2-{[3-(Benzyloxy)benzyl]oxy}-6-(1-piperazinyl)pyrazine.

### Step 1: 2-{[3-(Benzyloxy)benzyl]oxy}-6-chloropyrazine.

The title compound was prepared according to the procedure of example 50, step 1, starting from 3-benzyloxybenzyl alcohol (3.46 g, 16.2 mmol), K-*t-*BuO (1.69 g, 15.1 mmol) and 2,6-dichloropyrazine (1.97 g, 13.2 mmol). The yield of the title compound was 2.64 g (61%) and was obtained as a semisolid. Anal. (C₁₈H₁₅ClN₂O₂) C, H, N.

### Step 2: 2-{[3-(Benzyloxy)benzyl]oxy}-6-(1-piperazinyl)pyrazine.

The title compound was prepared according to the procedure of example 50, step 2, starting from the product obtained in step 1 above (1.62 g, 4.96 mmol), piperazine (1.28 g, 14.9 mmol) and K₂CO₃ (0.70 g, 5.1 mmol). The yield of the title compound was 1.16 g (62%) and was obtained as an oil. HRMS *m*/*z* calcd for C₂₂H₂₄N₄O₂ (M) ⁺ 376.1899, found 376.1890. Anal. (C₂₂H₂₄N₄O₂) C, H, N.

### EXAMPLE 67

### 2-(1-Piperazinyl)-6-[3-(2-pyridinyl)propoxy]pyrazine, Maleate.

### Step 1: 2-Chloro-6-[3-(2-pyridinyl)propoxy]pyrazine.

The title compound was prepared according to the procedure of example 50, step 1, starting from 2-pyridinepropanol (4.08 g, 29.7 mmol), K-*t-*BuO (3.17 g, 28.3 mmol) and 2,6-dichloropyrazine (3.69 g, 24.8 mmol). The yield of the title compound was 5.18 g (84%) and was obtained as an oil. Anal. (C₁₂H₁₂ClN₃O) C, H, N.

### Step 2: 2-(1-Piperazinyl)-6-[3-(2-pyridinyl)propoxy]pyrazine, Maleate.

The title compound was prepared according to the procedure of example 50, step 2, starting from the product obtained in step 1 above (1.80 g, 7.20 mmol), piperazine (1.87 g, 21.6 mmol) and K₂CO₃ (1.0 g, 7.2 mmol). The free base (1.23 g) of the title compound was converted into its maleate. Recrystallization from MeOH-ether furnished 1.32 g (38%) of the title compound. HRMS *m*/*z* calcd for C₁₆H₂₁N₅O (M)⁺ 299.1758, found 299.1748. Anal. (C₁₆H₂₁N₅O · 1.5C₄H₄O₄ · 0.5H₂O) C, H, N.

### EXAMPLE 68

### 2-[(3,5-Dimethoxybenzyl)oxy]-6-(1-piperazinyl)pyrazine, Maleate.

### Step 1: 2-Chloro-6-[(3,5-dimethoxybenzyl)oxy]pyrazine.

The title compound was prepared according to the procedure of example 50, step 1, starting from 3,5-dimethoxybenzyl alcohol (2.16 g, 12.8 mmol), K-*t-*BuO (1.34 g, 11.9 mmol) and 2,6-dichloropyrazine (1.59 g, 10.7 mmol). The yield of the title compound was 2.56 g (84%) and was obtained as a white solid. HRMS *m*/*z* calcd for C₁₃H₁₃ClN₂O₃ (M) ⁺ 280.0615, found 280.0627. Anal. (C₁₃H₁₃ClN₂O₃) C, H, N.

### Step 2: 2-[(3,5-Dimethoxybenzyl)oxy]-6-(1-piperazinyl)pyrazine, Maleate.

The title compound was prepared according to the procedure of example 50, step 2, starting from the product obtained in step 1 above (1.26 g, 4.50 mmol), piperazine (1.12 g, 13.0 mmol) and K₂CO₃ (0.62 g, 4.5 mmol). The free base (1.14 g) of the title compound was converted into its maleate salt. Recrystallization from MeOH-ether furnished 1.05 g (68%) of the title compound: mp 134-137 °C. HRMS *m*/*z* calcd for C₁₇H₂₂N₄O₃ (M)⁺ 330.1692, found 330.1699. Anal. (C₁₇H₂₂N₄O₃ · C₄H₄O₄) C, H, N.

### EXAMPLE 69

### 2-[2-(4-Methoxyphenyl)ethoxy]-6-(1-piperazinyl)pyrazine, Maleate.

### Step 1: 2-Chloro-6-[2-(4-methoxyphenyl)ethoxy]pyrazine.

The title compound was prepared according to the procedure of example 50, step 1, starting from 4-methoxyphenethyl alcohol (1.99 g, 13.1 mmol), K-*t-*BuO (1.34 g, 12.0 mmol) and 2,6-dichloropyrazine (1.56 g, 10.5 mmol). The yield of the title compound was 2.14 g (77%) and was obtained as a white solid. Anal. (C₁₃H₁₃ClN₂O₂) C, H, N.

### Step 2: 2-[2-(4-Methoxyphenyl)ethoxy]-6-(1-piperazinyl)pyrazine, Maleate.

The title compound was prepared according to the procedure of example 50, step 2, starting from the product obtained in step 1 above (1.31 g, 4.95 mmol), piperazine (1.24 g, 14.4 mmol) and K₂CO₃ (0.68 g, 4.9 mmol). The free base (1.29 g) of the title compound was converted into its maleate salt. Recrystallization from MeOH-ether furnished 1.41 g (79%) of the title compound: mp 149-151 °C. HRMS *m*/*z* calcd for C₁₇H₂₂N₄O₂ (M) ⁺ 314.1743, found 314.1727. Anal. (C₁₇H₂₂N₄O₂ · C₄H₄O₄) C, H, N.

### EXAMPLE 70

### 2-[2-(4-Methyl-1,3-thiazol-5-yl)ethoxy]-6-(1-piperazinyl)pyrazine, Acetate.

The title compound was prepared according to the procedure of example 42 starting from 4-methyl-5-hydroxyethyl thiazole (0.215 g, 1.50 mmol) and was isolated as a brown oil. Yield: 0.41 g (66%). HRMS *m*/*z* calcd for C₁₄H₁₉N₅OS (M)⁺ 305.1310, found 300.1325. Anal. (C₁₄H₁₉N₅OS · 1.5CH₃COOH · 0.7H₂O).

### General procedure for synthesis of the title compounds described in

### Examples 71-96.

To the appropriate alcohol or thiol (1.8 mmol) in dry DMF (5 mL) was added Na-*t-*BuO (1.20 ml, 2.5 M in DMF) and the mixture was stirred at room temperature for 15 minutes. To the mixture was added a solution of the appropriate piperazino-substituted chloro heterocycle (0.625 mL, 2.0 M in DMF) and the mixture were stirred at 100 °C for 5 h. The reactions were quenched with water (0.2 mL) and the solvent was removed under reduced pressure. The residue was taken up in water/CHCl₃ (20:80; 5 mL) and applied onto a column of Hydromatrix (40 mL) to which water (5 mL) had been added. Elution with CHCl₃ (4 x 8 mL) furnished the crude products. Concentration under reduced pressure and purification of the residues by preparative HPLC furnished the desired products as their acetic acid salts.

### EXAMPLE 71

### 2-[2-(3-Methoxyphenoxy)ethoxy]-6-(1-piperazinyl)pyrazine, Acetic acid salt.

Starting materials, 2-chloro-6-(1-piperazinyl)pyrazine*, and 2-(3-methoxyphenoxy)-ethanol. Purity 90% (HPLC). Fragmenting mass analysis supports the stated structure. HRMS *m*/*z* calcd for C₁₇H₂₂N₄O₃ (M)⁺ 330.1692, found 330.1681. *Obtained in Example 13, step 2.

### EXAMPLE 72

### 2-[2-(2,6-Difluorophenoxy)ethoxy]-6-(1-piperazinyl)pyrazine, Acetic acid salt.

Starting materials, 2-chloro-6-(1-piperazinyl)pyrazine and 2-(2,6-difluorophenoxy)-ethanol. Fragmenting mass analysis supports the stated structure. Purity 90% (HPLC). HRMS *m*/*z* calcd for C₁₆H₁₈F₂N₄O₃ (M)⁺ 336.1398, found 336.1403.

### EXAMPLE 73

### 2-[2-(Quinolin-8-yloxy)ethoxy]-6-(1-piperazinyl)pyrazine, Acetic acid salt.

Starting materials, 2-chloro-6-(1-piperazinyl)pyrazine and 2-(quinolin-8-yloxy)ethanol.*
Fragmenting mass analysis supports the stated structure. Purity 90% (HPLC). HRMS *m*/*z* calcd for C₁₉H₂₁N₅O₂ (M)⁺351.1695, found 351.1683. *Described in WO 00/76984.

### EXAMPLE 74

### 2-[(2R)-2,3-Dihydro-1,4-benzodioxin-2-ylmethoxy]-6-(1-piperazinyl)pyrazine, Acetic acid salt.

Starting materials, 2-chloro-6-(1-piperazinyl)pyrazine and (2*R*)-2-hydroxymethyl-1,4-benzodioxan*. Fragmenting mass analysis supports the stated structure. Purity 90% (HPLC). HRMS *m*/*z* calcd for C₁₇H₂₀N₄O₃ (M)⁺ 328.1535, found 328.1524.
*Described in Tetrahedron Lett. 1988, 29, 3671-4.

### EXAMPLE 75

### 2-[2-(2-Naphthyloxy)ethoxy]-6-(1-piperazinyl)pyrazine, Acetic acid salt.

Starting materials, 2-chloro-6-(1-piperazinyl)pyrazine and 2-(naphthaten-2-yloxy)-ethanol. Fragmenting mass analysis supports the stated structure. Purity 90% (HPLC). HRMS *m*/*z* calcd for C₂₀H₂₂N₄O₂ (M)⁺ 350.1743, found 350.1752

### EXAMPLE 76

**2-{2-[(2-Ethoxy-3-pyridinyl)oxy]ethoxy}-6-(1-piperazinyl)pyrazine, Acetic acid salt**. Starting materials, 2-chloro-6-(1-piperazinyl)pyrazine and 2-(2-ethoxypyridin-3-yloxy)-ethanol*. Fragmenting mass analysis supports the stated structure. Purity 80% (HPLC). HRMS *m*/*z* calcd for C₁₇H₂₃N₅O₃ (M)⁺ 345.1801, found 345.1793.
*Described in WO 00/76984.

### EXAMPLE 77

**2-{[4-(Benzyloxy)-3-methoxybenzyl]oxy}-6-(1-piperazinyl)pyrazine, Acetic acid salt**. Starting materials, 2-chloro-6-(1-piperazinyl)pyrazine and (4-benzyloxy-3-methoxyphenyl)-methanol. Fragmenting mass analysis supports the stated structure. Purity 90% (HPLC). HRMS *m*/*z* calcd for C₂₃H₂₆N₄O₃ (M)⁺ 406.2005, found 406.1967.

### EXAMPLE 78

**2-{[5-(Phenylethynyl)-2-thienyl]methoxy}-6-(1-piperazinyl)pyrazine, Acetic acid salt**. Starting materials, 2-chloro-6-(1-piperazinyl)pyrazine and (5-phenylethynyl-thiophen-2-yl)-methanol. Fragmenting mass analysis supports the stated structure Purity 80% (HPLC). HRMS *m*/*z* calcd for C₂₁H₂₀N₄OS (M)⁺376.1358, found 376.1346.

### EXAMPLE 79

**2-(2,3-Dihydro-1,4-benzodioxin-6-ylmethoxy)-6-(1-piperazinyl)pyrazine, Acetic acid salt**. Starting materials, 2-chloro-6-(1-piperazinyl)pyrazine and (2,3-dihydro-1,4-benzodioxin-6-yl)-methanol. Fragmenting mass analysis supports the stated structure. Purity 90% (HPLC). HRMS *m*/*z* calcd for C₁₇H₂₀N₄O₃ (M)⁺ 328.1535, found 328.1543.

### EXAMPLE 80

### 2-(1-Methyl-2-phenylethoxy)-6-(1-piperazinyl)pyrazine, Acetic acid salt.

The general procedure was followed with the exception that the reaction mixture was heated at 100 °C overnight. Starting materials, 2-chloro-6-(1-piperazinyl)pyrazine and
1-phenylpropan-2-ol. Fragmenting mass analysis supports the stated structure. Purity 70% (HPLC). HRMS *m*/*z* calcd for C₁₇H₂₂N₄O (M)⁺ 298.1794, found 298.1801.

### EXAMPLE 81

### 2-[(2-Chlorobenzyl)sulfanyl]-6-(1-piperazinyl)pyrazine, Acetic acid salt.

Starting materials, 2-chloro-6-(1-piperazinyl)pyrazine and (2-chlorophenyl)-methanethiol. Fragmenting mass analysis supports the stated structure.
Purity 90% (HPLC). HRMS *m*/*z* calcd for C₁₅H₁₇CN₄S (M)⁺ 320.0862, found 320.0868

### EXAMPLE 82

### 2-[(2-Phenylethyl)sulfanyl]-6-(1-piperazinyl)pyrazine, Acetic acid salt.

Starting materials, 2-chloro-6-(1-piperazinyl)pyrazine and 2-phenyl-ethanethiol. Fragmenting mass analysis supports the stated structure. Purity 90% (HPLC). HRMS *m*/*z* calcd for C₁₆H₂₀N₄S (M)⁺ 300.1409, found 300.1419.

### EXAMPLE 83

### 2-[(4-Phenoxybenzyl)oxy]-6-(1-piperazinyl)pyrazine, Acetic acid salt.

Starting materials 2-chloro-6-(1-piperazinyl)pyrazine and 4-phenoxybenzyl alcohol*. Fragmenting mass analysis supports the stated structure. Purity 90% (HPLC). HRMS *m*/*z* calcd for C₂₁H₂₂N₄O₂ (M)⁺ 362.1743, found 362.1738. *Prepared by reduction of 4-phenoxybenzaldehyde.

### EXAMPLE 84

**2-{[4-(3-Dimethylamino-propoxy)benzyl]oxy}-6-(1-piperazinyl)pyrazine, Acetic acid salt.** Starting materials, 2-chloro-6-(1-piperazinyl)pyrazine and [4-(3-dimethylamino-propoxy)-phenyl]-methanol. Fragmenting mass analysis supports the stated structure. Purity 90% (HPLC). HRMS *m*/*z* calcd for C₂₀H₂₉N₅O₂ (M)⁺ 371.2321, found 371.2314.

### EXAMPLE 85

### 2-{2-[2-(Benzyloxy)phenyl]ethoxy}-6-(1-piperazinyl)pyrazine, Acetic acid salt.

Starting materials, 2-chloro-6-(1-piperazinyl)pyrazine and 2-(2-benzyloxy-phenyl)-ethanol.* Fragmenting mass analysis supports the stated structure. Purity 70% (HPLC). HRMS *m*/*z* calcd for C₂₃H₂₆N₄O₂ (M)⁺ 390.2056, found 390.2043.
*Prepared by reduction of (2-benzyloxy-phenyl)-acetic acid.

### EXAMPLE 86

### 2-[2-(2,5-Dimethoxyphenyl)ethoxy]-6-(1-piperazinyl)pyrazine, Acetic acid salt.

Starting materials, 2-chloro-6-(1-piperazinyl)pyrazine and 2-(2,5-dimethoxyphenyl)-ethanol*. Fragmenting mass analysis supports the stated structure. Purity 80% (HPLC). HRMS *m*/*z* calcd for C₁₈H₂₄N₄O₃ (M)⁺ 344.1848, found 344.1861.
*Prepared by reduction of (2,5-dimethoxy-phenyl)-acetic acid.

### EXAMPLE 87

### 2-(1-Benzofuran-2-ylmethoxy)-6-(1-piperazinyl)pyrazine, Acetic acid salt.

Starting materials, 2-chloro-6-(1-piperazinyl)pyrazine and benzofuran-2-yl-methanol.*
Fragmenting mass analysis supports the stated structure. Purity 80% (HPLC). HRMS *m*/*z* calcd for C₁₇H₁₈N₄O₂ (M)⁺310.1430, found 310.1419. *Prepared by reduction of benzofuran-2-carbaldehyde.

### EXAMPLE 88

### 2-{2-[3-Methoxy-2-(phenoxymethyl)phenyl] ethoxy}-6-(1-piperazinyl)pyrazine,

**Acetic acid salt**. Starting materials, 2-chloro-6-(1-piperazinyl)pyrazine and (3-methoxy-2-phenoxymethyl-phenyl)-methanol.* Fragmenting mass analysis supports the stated structure. Purity 90% (HPLC). HRMS *m*/*z* calcd for C₂₃H₂₆N₄O₃ (M)⁺ 406.2005, found 406.2011. *Prepared by reduction of 3-methoxy-2-phenoxymethyl-benzaldehyde.

### EXAMPLE 89

### 2-[2-(Isoquinolin-7-yloxy)ethoxy]-6-(1-piperazinyl)pyrazine, Acetic acid salt.

### Step 1: 2-(7-Isoquinolinyloxy)ethanol.

A mixture of 7-hydroxyisoquinoline (1.15 g, 7.9 mmol), ethylene carbonate (0.98 g, 11.1 mmol), powdered K₂CO₃ (0.65 g, 4.7 mmol) in dry DMF (20 mL) was stirred at 145 °C for two hours. The reaction was quenched with MeOH (1 mL), filtered and the solvent was removed under reduced pressure. The residue was taken up between alkaline water (K₂CO₃) and CHCl₃. Concentration of the dried (MgSO₄) organic phase afforded 1.4 g (94%) of the title compound as a yellow oil that solidified upon standing. Purity 91% (HPLC). Fragmenting mass analysis supports the stated structure.

**Step 2: 2-[2-(Isoquinolin-7-yloxy)ethoxy]-6-(1-piperazinyl)pyrazine, Acetic acid salt.** The general procedure was followed starting 2-chloro-6-(1-piperazinyl)pyrazine and 2-(7-isoquinolinyloxy)ethanol. Fragmenting mass analysis supports the stated structure. Purity 80% (HPLC). HRMS *m*/*z* calcd for C₁₉H₂₁N₅O₂ (M)⁺ 351.1695, found 351.1696.

### EXAMPLE 90

### 2-(2,3-Dihydro-1H-inden-2-yloxy)-6-(1-piperazinyl)pyrazine, Acetic acid salt.

Starting materials, 2-chloro-6-(1-piperazinyl)pyrazine and 2-indanol. The general procedure was followed with the exception that the reaction mixture was heated at 100 °C overnight. Fragmenting mass analysis supports the stated structure. Purity 90% (HPLC). HRMS *m*/*z* calcd for C₁₇H₂₀N₄O (M)⁺296.1637, found 296.1652.

### EXAMPLE 91

### 2-{[2-(Phenoxymethyl)benzyl]oxy}-6-(1-piperazinyl)pyrazine, Acetic acid salt.

Starting materials, 2-chloro-6-(1-piperazinyl)pyrazine and 2-phenoxymethyl-benzyl alcohol.* Fragmenting mass analysis supports the stated structure. Purity 90% (HPLC). HRMS *m*/*z* calcd for C₂₂H₂₄N₄O₂ (M)⁺376.1899, found 376.1889.
*Prepared by reduction of 2-phenoxymethylbenzoic acid by lithium aluminum hydride in THF, *cf* J. Chem. Soc. 1954, 2819.

### EXAMPLE 92

### 2-(2-Cyclohexylethoxy)-6-(1-piperazinyl)pyrazine, Acetic acid salt.

Starting materials, 2-chloro-6-(1-piperazinyl)pyrazine and 2-cyclohexyl-ethanol. Fragmenting mass analysis supports the stated structure. Purity 90% (HPLC). HRMS *m*/*z* calcd for C₁₆H₂₆N₄O (M)⁺ 290.2107, found 290.2109.

### EXAMPLE 93

### 2-[2-(2-Amino-quinolin-8-yloxy)ethoxy]-6-(1-piperazinyl)pyrazine, Acetic acid salt.

Starting materials, 2-chloro-6-(1-piperazinyl)pyrazine and 2-(2-amino-quinolin-8-yloxy)-ethanol.* Fragmenting mass analysis supports the stated structure.
Purity 90% (HPLC). HRMS *m*/*z* calcd for C₁₉H₂₂N₆O₂ (M)⁺ 366.1804, found 366.1791. *Prepared as described in WO 00/76984.

### EXAMPLE 94

### 2-[(3-Cyanobenzyl)oxy]-6-(1-piperazinyl)pyrazine.

Starting materials, 2-chloro-6-(1-piperazinyl)pyrazine and 3-cyanobenzyl alcohol. Fragmenting mass analysis supports the stated structure. Purity 90% (HPLC). HRMS *m*/*z* calcd for C₁₆H₁₇N₅O (M)⁺ 295.1433, found 295.1431.

### EXAMPLE 95

### 2-[(5-Fluoro-2-methoxybenzyl)oxy]-6-(1-piperazinyl)pyrazine, Acetic acid salt.

Starting materials, 2-chloro-6-(1-piperazinyl)pyrazine and (5-fluoro-2-methoxyphenyl)-methanol.* Fragmenting mass analysis supports the stated structure. Purity 90% (HPLC). HRMS *m*/*z* calcd for C₁₆H₁₉FN₄O₂ (M)⁺318.1492, found 318.1490.
*Prepared by reduction of 5-fluoro-2-methoxybenzaldehyde.

### EXAMPLE 96

### 2-(1-Cyclopentylethoxy)-6-(1-piperazinyl)pyrazine, Acetic acid salt.

Starting materials, 2-chloro-6-(1-piperazinyl)pyrazine and 1-cyclopentyl-ethanol. The general procedure was followed with the exceptions that dioxane was used as solvent and that the reaction mixture was heated in a sealed tube with microwaves at 160 °C for 20 minutes. Fragmenting mass analysis supports the stated structure. Purity 90% (HPLC). HRMS *m*/*z* calcd for C₁₅H₂₄N₄O (M)⁺ 276.1950, found 276.1955.

### EXAMPLE 97

### 2-[(2,5-Difluorobenzyl)oxy]-6-(1-piperazinyl)pyrazine, Maleate.

The title compound was prepared according to the procedure of example 50, step 2, starting from 2-chloro-6-[(2,5-difluorobenzyl)oxy]pyrazine (3.43 g, 13.4 mmol; obtained according to the procedure of example 50, step 1, starting from 2,5-difluorobenzyl alcohol), piperazine (3.51 g, 40.7 mmol) and K₂CO₃ (1.94 g, 14.0 mmol) with the exception that the final filtration through alumina was omitted. The yield of the free base of the title compound was 2.84 g (69%) which was obtained as an oil. The free base was converted into its maleate salt. Purity 100% (HPLC). MS *m*/*z* 306 (M)⁺. HRMS *m*/*z* calcd for C₁₅H₁₆F₂N₄O (M)⁺ 306.1292, found 306.1297.

### EXAMPLE 98

### 2-[(3-Dimethylaminobenzyl)oxy]-6-(1-piperazinyl)pyrazine.

The title compound was prepared according to the procedure of example 50, step 2, starting from 2-chloro-6-[(3-dimethylaminobenzyl)oxy]pyrazine (3.04 g, 11.5 mmol; obtained according to the procedure of example 50, step 1, starting from 3-dimethylaminobenzyl alcohol), piperazine (3.08 g, 35.7 mmol) and K₂CO₃ (1.59 g, 11.5 mmol) with the exception that the final filtration through alumina was omitted. The yield of the of the title compound was 2.06 g (57%) which was obtained as a beige colored oil that solidified when refrigerated. Purity 98% (HPLC). MS *mlz* 313 (M)⁺. HRMS *m*/*z* calcd for C₁₇H₂₃N₅O (M)⁺ 313.1903, found 313.1910.

### EXAMPLE 99

### 2-[{4-(2-Pyridinyl)benzyl}oxy]-6-(1-piperazinyl)pyrazine.

The title compound was prepared according to the procedure of example 50, step 2, starting from 2-chloro-6-[{4-(2-pyridinyl)benzyl}oxy]pyrazine (2.73 g, 9.16 mmol; obtained according to the procedure of example 50, step 1, starting from 4-(2-pyridinyl)benzyl alcohol*), piperazine (2.41 g, 27.9 mmol) and K₂CO₃ (1.33 g, 9.62 mmol) with the exception that the final filtration through alumina was omitted. The yield of the of the title compound was 2.06 g (65%) which was obtained as a beige colored oil that solidified when refrigerated. Purity 100% (HPLC). MS *m*/*z* 347 (M)⁺. HRMS *m*/*z* calcd for C₂₀H₂₁N₅O (M)⁺ 347.1746, found 347.1749. *Obtained by reduction (NaBH₄) of 4-(2-pyridyl)benzaldehyde.

### EXAMPLE 100

### 2-[(2-Fluorobenzyl)oxy]-6-(1-piperazinyl)pyrazine, Maleate.

The title compound was prepared according to the procedure of example 50, step 2, starting from 2-chloro-6-[(2-fluorobenzyl)oxy]pyrazine (3.68 g, 15.4 mmol; obtained according to the procedure of example 50, step 1, starting from 2-fluorobenzyl alcohol), piperazine (4.06 g, 47.1 mmol) and K₂CO₃ (2.24 g, 16.2 mmol) with the exception that the final filtration through alumina was omitted. The yield of the free base of the title compound was 3.28 g (74%) which was obtained as an oil. The free base was converted into its maleate salt. Purity 100% (HPLC). MS *m*/*z* 288 (M)⁺. HRMS *m*/*z* calcd for C₁₅H₁₇FN₄O (M)⁺ 288.1386, found 288.1378.

### EXAMPLE 101

### 2-(Benzo[b]thiophen-3-ylmethoxy)-6-(1-piperazinyl)pyrazine, Maleate.

The title compound was prepared according to the procedure of example 50, step 2, starting from 2-chloro-6-(benzo[*b*]thiophen-3-ylmethoxy)pyrazine (2.88 g, 10.4 mmol; obtained according to the procedure of example 50, step 1, starting from benzo[*b*]thiophene-3-methanol), piperazine (2.73 g, 31.7 mmol) and K₂CO₃ (1.51 g, 10.9 mmol) with the exception that the final filtration through alumina was omitted. The yield of the free base of the title compound was 2.34 g (69%) which was obtained as a beige colored oil. The free base was converted into its maleate salt. Purity 99% (HPLC). MS *m*/*z* 326 (M)⁺. HRMS *m*/*z* calcd for C₁₇H₁₈N₄OS (M)⁺ 326.1201, found 326.1207.

### EXAMPLE 102

### 2-(3-Phenoxy-thiophen-2-ylmethoxy)-6-(1-piperazinyl)pyrazine, Maleate.

The title compound was prepared according to the procedure of example 50, step 2, starting from 2-chloro-6-(3-phenoxy-thiophen-2-ylmethoxy)pyrazine [2.83 g, 8.88 mmol; obtained according to the procedure of example 50, step 1, starting from (3-phenoxy-2-thienyl)methanol], piperazine (2.33 g, 27.1 mmol) and K₂CO₃ (1.29 g, 9.3 mmol) with the exception that the final filtration through alumina was omitted. The yield of the free base of the title compound was 1.80 g (55%) which was obtained as a beige colored oil. The free base was converted into its maleate salt. Purity 98% (HPLC). MS *m*/*z* 368 (M)⁺. HRMS *m*/*z* calcd for C₁₉H₂₀N₄O₂S (M)⁺ 368.1307, found 368.1306.

### EXAMPLE 103

### 2-[5-(2-Pyridinyl)-thiophen-2-ylmethoxy)]-6-(1-piperazinyl)pyrazine, Maleate.

The title compound was prepared according to the procedure of example 50, step 2, starting from 2-chloro-6-[5-(2-pyridinyl)-thiophen-2-ylmethoxy)]pyrazine [2.17 g, 7.13 mmol; obtained according to the procedure of example 50, step 1, starting from 5-(pyridin-2-yl)thiophene-2-methanol], piperazine (1.84 g, 21.4 mmol) and K₂CO₃ (0.99 g, 7.1 mmol) with the exception that the final filtration through alumina was omitted. The yield of the free base of the title compound was 1.66 g (66%) which was obtained as a beige colored oil. The free base was converted into its maleate salt. Purity 100% (HPLC). MS *m*/*z* 353 (M)⁺. HRMS *m*/*z* calcd for C₁₈H₁₉N₅OS (M)⁺ 353.1310, found 353.1307.

### EXAMPLE 104

### 2-[2-(5-Methyl-2-phenyl-oxazol-4-yl)-ethoxy]-6-(1-piperazinyl)pyrazine.

The title compound was prepared according to the procedure of example 50, step 2, starting from 2-chloro-6-[2-(5-methyl-2-phenyl-oxazol-4-yl)-ethoxy]pyrazine [2.90 g, 9.18 mmol; obtained according to the procedure of example 50, step 1, starting from 2-(5-methyl-2-phenyloxazol-4-yl)ethanol], piperazine (2.37 g, 27.5 mmol) and K₂CO₃ (1.27 g, 9.19 mmol) with the exception that the final filtration through alumina was omitted. The yield of the of the title compound was 2.09 g (62%) which was obtained as a light yellow oil that solidified when refrigerated. Purity 100% (HPLC). MS *m*/*z* 365 (M)⁺. HRMS *m*/*z* calcd for C₂₀H₂₃N₅O₂ (M)⁺ 365.1852, found 365.1855.

### EXAMPLE 105

### 2-[1-(2,6-Difluoro-phenyl)-ethoxy]-6-(1-piperazinyl)pyrazine, Maleate.

The title compound was prepared according to the procedure of example 50, step 2, starting from 2-chloro-6-[1-(2,6-difluoro-phenyl)-ethoxy]pyrazine (3.20 g, 11.8 mmol; obtained according to the procedure of example 50, step 1, starting from 2,6-difluoro-α-methylbenzyl alcohol), piperazine (3.05 g, 35.4 mmol) and K₂CO₃ (1.63 g, 11.8 mmol) with the exception that the final filtration through alumina was omitted. The yield of the free base of the title compound was 2.95 g (78%) which was obtained as a colorless oil. The free base was converted into its maleate salt. Purity 100% (HPLC). MS *m*/*z* 320 (M)⁺. HRMS *m*/*z* calcd for C₁₆H₁₈F₂N₄O (M)⁺ 320.1449, found 320.1447.

### EXAMPLE 106

### 2-(2-Naphthalen-2-yl-ethoxy)-6-(1-piperazinyl)pyrazine, Maleate.

The title compound was prepared according to the procedure of example 50, step 2, starting from 2-chloro-6-(2-naphthalen-2-yl-ethoxy)pyrazine (2.73 g, 9.60 mmol; obtained according to the procedure of example 50, step 1, starting from 2-naphthalene-ethanol), piperazine (2.89 g, 33.5 mmol) and K₂CO₃ (1.39 g, 10.1 mmol) with the exception that the final filtration through alumina was omitted. The yield of the free base of the title compound was 2.63 g (82%) which was obtained as a colorless oil. The free base was converted into its maleate salt. Purity 99% (HPLC). MS *m*/*z* 334 (M)⁺. HRMS *m*/*z* calcd for C₂₀H₂₂N₄O (M)⁺ 334.1794, found 334.1794.

### EXAMPLE 107

### 2-[3-(Naphthalen-2-yloxy)-propoxy]-6-(1-piperazinyl)pyrazine, Maleate.

The title compound was prepared according to the procedure of example 50, step 2, starting from 2-chloro-6-[3-(naphthalen-2-yloxy)-propoxy]pyrazine [2.24 g, 7.12 mmol; obtained according to the procedure of example 50, step 1, starting from 3-(naphthalen-2-yloxy)-propan-1-ol*], piperazine (1.90 g, 22.1 mmol) and K₂CO₃ (1.03 g, 7.45 mmol) with the exception that the final filtration through alumina was omitted. The yield of the free base of the title compound was 1.10 g (42%) which was obtained as a light beige colored oil. The free base was converted into its maleate salt. Purity 100% (HPLC). MS *m*/*z* 364 (M)⁺. HRMS *m*/*z* calcd for C₂₁H₂₄N₄O₂ (M)⁺ 364.1899, found 364.1895. *Described in J. Am. Chem Soc. 1929, 51, 3417 and ibid. 1954, 76, 56.

### EXAMPLE 108

### 2-(4-Phenylethynyl-thiophen-2-ylmethoxy)-6-(1-piperazinyl)pyrazine, Maleate.

The title compound was prepared according to the procedure of example 50, step 2, starting from 2-chloro-6-(4-phenylethynyl-thiophen-2-ylmethoxy)pyrazine [2.05 g, 6.28 mmol; obtained according to the procedure of example 50, step 1, starting from 4-(phenylethynyl)thiophene-2-methanol*], piperazine (1.62 g, 18.8 mmol) and K₂CO₃ (0.89 g, 6.4 mmol) with the exception that the final filtration through alumina was omitted. The yield of the free base of the title compound was 1.80 g (76%) which was obtained as a light beige colored oil. The free base was converted into its maleate salt. Purity 100% (HPLC). MS *m*/*z* 376 (M)⁺. HRMS *m*/*z* calcd for C₂₁H₂₀N₄OS (M)⁺ 376.1358, found 376.1351. *Obtained by reduction (NaBH₄) of 4-(phenylethynyl)thiophene-2-carboxaldehyde.

### EXAMPLE 109

### 2-(1-Cyclopropyl-ethoxy)-6-(1-piperazinyl)pyrazine, Maleate.

The title compound was prepared according to the procedure of example 50, step 2, starting from 2-chloro-6-(1-cyclopropyl-ethoxy)pyrazine (2.38 g, 12.0 mmol; obtained according to the procedure of example 50, step 1, starting from α-methylcyclopropanemethanol), piperazine (3.60 g, 41.8 mmol) and K₂CO₃ (1.75 g, 12.7 mmol) with the exception that the final filtration through alumina was omitted. The yield of the free base of the title compound was 2.05 g (69%) which was obtained as a colorless oil. The free base was converted into its maleate salt. Purity 100% (HPLC). MS *m*/*z* 248 (M)⁺. HRMS *m*/*z* calcd for C₁₃H₂₀N₄O (M)⁺ 248.1637, found 248.1636.

### EXAMPLE 110

### 2-[2-(6-Methoxy-naphthalen-2-yloxy)-ethoxy]-6-(1-piperazinyl)pyrazine.

The title compound was prepared according to the procedure of example 50, step 2, starting from 2-chloro-6-[2-(6-methoxy-naphthalen-2-yloxy)-ethoxy]pyrazine [0.94 g, 2.8 mmol; obtained according to the procedure of example 50, step 1, starting from 2-(6-methoxy-naphthalen-2-yloxy)ethanol*], piperazine (1.00 g, 11.6 mmol) and K₂CO₃ (0.50 g, 3.6 mmol) with the exception that the final filtration through alumina was omitted. The yield of the of the title compound was 0.52 g (48%) which was obtained as a beige colored solid. Purity 100% (HPLC). MS *m*/*z* 380 (M)⁺. HRMS *m*/*z* calcd for C₂₁H₂₄N₄O₃ (M)⁺ 380.1848, found 380.1845. *Prepared from 6-methoxy-2-naphthol and ethylene carbonat according to the procedure of Example 134, step 1, in WO 00/76984. The reaction mixture was refluxed for 2 h. Pure 2-(6-methoxy-naphthalen-2-yloxy)ethanol was obtained by recrystallization from MeOH/CHCl₃/*n*-hexane.

### EXAMPLE 111

### 2-[2-(7-Methoxy-naphthalen-2-yloxy)-ethoxy]-6-(1-piperazinyl)pyrazine.

The title compound was prepared according to the procedure of example 50, step 2, starting from 2-chloro-6-[2-(7-methoxy-naphthalen-2-yloxy)-ethoxy]pyrazine [1.19 g, 3.60 mmol; obtained according to the procedure of example 50, step 1, starting from 2-(7-methoxy-naphthalen-2-yloxy)ethanol*], piperazine (1.25 g, 14.5 mmol) and K₂CO₃ (0.60 g, 4.3 mmol) with the exception that the final filtration through alumina was omitted. The yield of the of the title compound was 0.98 g (71%) which was obtained as an oil that solidified when refrigerated. Purity 100% (HPLC). MS *m*/*z* 380 (M)⁺. HRMS *m*/*z* calcd for C₂₁H₂₄N₄O₃ (M)⁺ 380.1848, found 380.1851.
*Prepared from 7-methoxy-2-naphthol and ethylene carbonat according to the procedure of Example 134, step 1, in WO 00/76984. The reaction mixture was refluxed for 2 h. Pure 2-(7-methoxy-naphthalen-2-yloxy)ethanol was obtained after column chromatography on silica gel using *n*-hexane/ethyl acetate (6:4) as eluent.

### EXAMPLE 112

### 2-[5-(4-Chlorophenyl)-2-methylfuran-3-ylmethoxy]-6-(1-piperazinyl)pyrazine.

The title compound was prepared according to the procedure of example 50, step 2, starting from 2-chloro-6-[5-(4-chlorophenyl)-2-methylfuran-3-ylmethoxy]pyrazine [3.14 g, 9.39 mmol; obtained according to the procedure of example 50, step 1, starting from 5-(4-chlorophenyl)-3-hydroxymethyl-2-methylfuran], piperazine (2.47 g, 28.6 mmol) and K₂CO₃ (1.36 g, 9.86 mmol) with the exception that the final filtration through alumina was omitted. The yield of the of the title compound was 2.11 g (58%) which was obtained as a beige colored solid. Purity 100% (HPLC). MS *m*/*z* 384 (M)⁺. HRMS *m*/*z* calcd for C₂₀H₂₁ClN₄O₂ (M)⁺ 384.1353, found 384.1357.

### EXAMPLE 113

### 2-(1H-Indol-4-ylmethoxy)-6-(1-piperazinyl)pyrazine, Maleate.

The title compound was prepared according to the procedure of example 50, step 2, starting from 2-chloro-6-(1*H-*indol-4-ylmethoxy)pyrazine [0.486 g, 1.87 mmol; obtained according to the procedure of example 50, step 1, starting from (1*H-*indol-4-yl)-methanol*], piperazine (0.491 g, 5.71 mmol) and K₂CO₃ (0.272 g, 1.96 mmol) with the exception that the final filtration through alumina was omitted. The yield of the free base of the title compound was 0.198 g (34%) which was obtained as an oil. The free base was converted into its maleate salt. Purity 100% (HPLC). MS *m*/*z* 309 (M)⁺. HRMS *m*/*z* calcd for C₁₇H₁₉N₅O(M)⁺ 309.1590, found 309.1582. *The reaction was carried out using (1*H-*indol-4-yl)-methanol (0.712 g, 4.84 mmol), K-*t-*BuO (0.517 g, 4.61 mmol), and 2,6-dichloropyrazine (0.687 g, 4.61 mmol).

### EXAMPLE 114

### 2-(2-Phenyl-propoxy)-6-(1-piperazinyl)pyrazine, Maleate.

The title compound was prepared according to the procedure of example 50, step 2, starting from 2-chloro-6-(2-phenyl-propoxy)pyrazine (2.39 g, 9.61 mmol; obtained according to the procedure of example 50, step 1, starting from 2-phenyl-1-propanol), piperazine (2.90 g, 3.36 mmol) and K₂CO₃ (1.40 g, 10.1 mmol) with the exception that the final filtration through alumina was omitted. The yield of the free base of the title compound was 1.66 g (58%) which was obtained as a colorless oil. The free base was converted into its maleate salt. Purity 99% (HPLC). HRMS *m*/*z* calcd for C₁₇H₂₂N₄O (M)⁺ 298.1794, found 298.1795.

### EXAMPLE 115

### 2-[2-(2-Methoxyphenyl)ethoxy]-6-(1-piperazinyl)pyrazine, Maleate.

The title compound was prepared according to the procedure of example 50, step 2, starting from 2-chloro-6-[2-(2-methoxyphenyl)ethoxy]pyrazine (0.967 g, 3.65 mmol; obtained according to the procedure of example 50, step 1, starting from 2-methoxyphenethyl alcohol), piperazine (0.913 g, 10.6 mmol) and K₂CO₃ (0.505 g, 3.65 mmol). The yield of the free base of the title compound was 0.63 g (55%) which was obtained as a colorless oil. The free base was converted into its maleate salt. Purity 100% (HPLC). MS *m*/*z* 314 (M)⁺. HRMS *m*/*z* calcd for C₁₇H₂₂N₄O₂ (M)⁺ 314.1743, found 314.1750.

### EXAMPLE 116

### 2-[2-(3-Methoxyphenyl)ethoxy]-6-(1-piperazinyl)pyrazine.

The title compound was prepared according to the procedure of example 50, step 2, starting from 2-chloro-6-[2-(3-methoxyphenyl)ethoxy]pyrazine (1.12 g, 4.23 mmol; obtained according to the procedure of example 50, step 1, starting from 3-methoxyphenethyl alcohol), piperazine (1.06 g, 12.3 mmol) and K₂CO₃ (0.585 g, 4.23 mmol). The yield of the title compound was 0.91 g (69%) which was obtained as light beige oil. HRMS *m*/*z* calcd for C₁₇H₂₂N₄O₂ (M)⁺ 314.1743, found 314.1759. Anal. (C₁₇H₂₂N₄O₂) C, H, N.

### EXAMPLE 117

### 2-[(2-Phenoxybenzyl)oxy]-6-(1-piperazinyl)pyrazine, Maleate.

The title compound was prepared according to the procedure of example 50, step 2, starting from 2-chloro-6-[(2-phenoxybenzyl)oxy]pyrazine (0.981 g, 3.14 mmol; obtained according to the procedure of example 50, step 1, starting from 2-phenoxybenzyl alcohol), piperazine (0.784 g, 9.10 mmol) and K₂CO₃ (0.434 g, 3.14 mmol) with the exception that the final filtration through alumina was omitted. The yield of the free base of the title compound was 0.80 g (70%) which was obtained as a colorless oil. The free base was converted into its maleate salt. Anal. (C₂₁H₂₂N₄O₂· C₄H₄O₄) C, H, N.

### EXAMPLE 119

### (2R)-1-[6-{(2-Chlorobenzyl)sulfanyl}-2-pyrazinyl]-2-methylpiperazine, Hydrochloride.

Na-*t-*BuO (8.7 mmol 0.84 g) was added to a solution of 2-chlorobenzylthiol (5.7 mmol 0.90 g) in dry DMF (25 mL) and the mixture was stirred at room temperature for 10 minutes. (2*R*)-1-(6-chloro-2-pyrazinyl)-2-methylpiperazine (0.92 g, 4.35 mmol; obtained in Example 62, step 2) was added and the reaction was stirred at 70 °C for 2 h. The mixture was filtered through a plug of silica and the solvent from the filtrate was evaporated at reduced pressure. The brown residue was chromatographed over silica gel using CHCl₃/MeOH/aq NH₃ 90/10/0.25 as eluent. This furnished the free base of the title compound as a yellow oil. The free base was precipitated as its HCl salt with HCl/ether to give 1.10 g (68%) of the title compound as light yellow crystals. Purity 98% (HPLC). HRMS *m*/*z* for C₁₆H₁₉ClN₄S (M)⁺ calcd for 334.1019, found 334.1036.

### EXAMPLE 120

### 2-(3-Thienylmethoxy)-6-(1-piperazinyl)pyrazine.

The title compound was prepared according to the procedure of Example 20 starting from 3-thiophenemethanol (6.05 g, 53.0 mmol), K-*t-*BuO (0.897 g, 7.99 mmol) and 6-chloro-2-(1-piperazinyl)pyrazine (0.845 g, 4.25 mmol: obtained in Example 13, step 2). The reaction mixture was stirred at 105 °C for 7.5 h. Following chromatography on silica, solvents were evaporated off. The remaining oil was redissolved in ethyl acetetate and filtered through a short plug of alumina (5 x 3 cm) using ether/MeOH (96:4) as eluent. Solvent removal *in vacuo* gave 0.76 g (64%) of the title compound as a colorless oil. HRMS *m*/*z* for C₁₃H₁₆N₄OS (M)⁺ calcd for 276.1045, found 276.1037. Anal. (C₁₃H₁₆N₄OS · 0.25 H₂O) C, H, N.

### EXAMPLE 121

### 2-(3-Phenoxypropoxy)-6-(1-piperazinyl)pyrazine, Maleate.

The title compound was prepared according to the procedure of example 50, step 2, starting from 2-chloro-6-(3-phenoxypropoxy)pyrazine (1.04 g, 3.93 mmol; obtained according to the procedure of example 50, step 1, starting from 3-phenoxy-1-propanol), piperazine (0.981 g, 11.4 mmol) and K₂CO₃ (0.543 g, 3.93 mmol). Following chromatography on silica, solvents were evaporated off. The semi-solid residue (0.83 g) was redissolved in CHCl₃ and filtered. The clear solution was concentrated *in vacuo* and the resulting free base of the title compound was converted into its maleate salt. Yield: 0.90 g (53%). HRMS *mlz* calcd for C₁₇H₂₂N₄O₂ (M)⁺ 314.1743, found 314.1728. Anal. (C₁₇H₂₂N₄O₂ · C₄H₄O₄) C, H, N.

### EXAMPLE 122

### 2-{[4-(Benzyloxy)benzyl]oxy}-6-(1-piperazinyl)pyrazine.

The title compound was prepared according to the procedure of example 50, step 2, starting from 2-chloro-6-{[4-(benzyloxy)benzyl]oxy}pyrazine (1.15 g, 3.52 mmol; obtained according to the procedure of example 50, step 1, starting from 4-benzyloxybenzyl alcohol), piperazine (0.894 g, 10.4 mmol) and K₂CO₃ (0.486 g, 3.52 mmol). The yield of the title compound was 0.57 g (43%) which was obtained as a colorless viscous oil that solidified upon standing. Fragmenting mass analysis supports the stated structure. HRMS *m*/*z* calcd for C₂₂H₂₄N₄O₂ (M)⁺ 376.1899, found 376.1892.

### EXAMPLE 123

### 2-(n-Hexyioxy)-6-(1-piperazinyl)pyrazine.

The title compound was prepared according to the procedure of example 50, step 2, starting from 2-chloro-6-(*n*-hexyloxy)pyrazine (1.54 g, 7.17 mmol; obtained according to the procedure of example 50, step 1, starting from *n*-hexanol), piperazine (1.90 g, 22.1 mmol) and K₂CO₃ (0.99 g, 7.16 mmol). The yield of the title compound was 1.21 g (64%) which was obtained as a colorless oil. HRMS *m*/*z* calcd for C₁₄H₂₄N₄O (M)⁺ 264.1950, found 264.1953. Anal. (C₁₄H₂₄N₄O) C, H, N.

### EXAMPLE 124

### 2-(Propargyloxy)-6-(1-piperazinyl)pyrazine, Maleate.

The title compound was prepared according to the procedure of example 50, step 2, starting from 2-chloro-6-(propargyloxy)pyrazine (1.70 g, 10.1 mmol; obtained according to the procedure of example 50, step 1, starting from propargyl alcohol), piperazine (1.91 g, 22.2 mmol) and K₂CO₃ (1.39 g, 10.1 mmol) with the exception that the final filtration through alumina was omitted. Following repeated chromatography on silica, solvents were evaporated off. The yield of the free base of the title compound was 0.48 g (22%) which was obtained as a beige oil. The free base was converted into its maleate salt. HRMS *m*/*z* calcd for C₁₁H₁₄N₄O (M)⁺ 218.1168, found 218.1158. Anal. (C₁₁H₁₄N₄O · C₄H₄O₄) C, H, N.

### PREPARATION OF PHARMACEUTICAL COMPOSITIONS

### EXAMPLE: Preparation of tablets

| | Ingredients | mg/tablet |
|---|---|---|
| 1. | Active compound | 10.0 |
| 2. | Cellulose, microcrystalline | 57.0 |
| 3. | Calcium hydrogen phosphate | 15.0 |
| 4. | Sodium starch glycolate | 5.0 |
| 5. | Silicon dioxide, colloidal | 0.25 |
| 6. | Magnesium stearate | 0.75 |

The active ingredient 1 is mixed with ingredients 2, 3, 4 and 5 for about 10 minutes. The magnesium stearate is then added, and the resultant mixture is mixed for about 5 minutes and compressed into tablet form with or without film-coating.

### Pharmacological tests

The ability of a compound of the invention to bind or act at specific 5-HT receptor subtypes can be determined using *in vitro* and *in vivo* assays known in the art. The biological activity of compounds prepared in the Examples was tested using different tests.

### Affinity assay

The 5-HT_{2c} receptor affinity of compounds in the Examples was determined in competition experiments, where the ability of each compound in serial dilution to displace ³H-labelled 5-HT, bound to membranes prepared from a transfected HEK293 cell line stably expressing the human 5-HT_{2c} receptor protein, was monitored by Scintillation Proximity Assay technology. Non-specific binding was defined using 5 µM mianserin. Results obtained for exemplary compounds of the invention are illustrated in Table 1 below. Typically, the 5-HT_{2C} receptor affinity values (Kᵢ, nM) were in the range of 1 nM to 1500 nM.

**Table 1. 5-HT_{2C} receptor Affinity**

| Compound | Kᵢ (nM) |
|---|---|
| Example 2 | 8 |
| Example 12 | 197 |
| Example 15 | 616 |
| Example 20 | 28 |
| Example 23 | 478 |

### Efficacy assay

The agonist efficacy at the 5-HT_{2c} receptor of the compounds in the Examples was determined by the ability of each compound to mobilise intracellular calcium in transfected HEK293 cells, stably expressing the human 5-HT_{2c} receptor protein, using the calcium-chelating fluorescent dye FLUO-3 (Sigma, St. Louis, MO, U.S.A.).

Typically, the maximum responses of 5-HT_{2c} agonists were in the range of 20-100% relative to the maximum response of 5-HT (serotonin) at a concentration of 1 µM.

## Claims

1. The compound of the general formula (II): 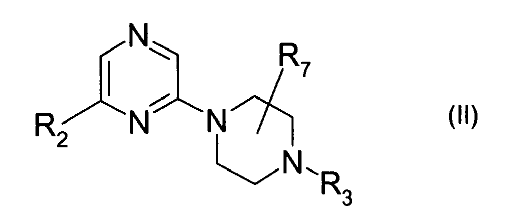 wherein
R₂ is selected from the group consisting of aryl-C₁-C₆-alkyl, aryl-C₁-C₆-alkoxy, heteroaryl-C₁-C₆-alkoxy, aryloxy-C₂-C₆-alkoxy, heteroaryloxy-C₂-C₆-alkoxy, 1-indanyloxy, 2-indanyloxy, aryloxy, heteroaryloxy, arylthio, heteroarylthio, C₅-C₆-cycloalkylthio, C₅-C₈-alkoxy, C₅-C₈-alkylthio, C₃-C₆-alkynyloxy, C₃-C₆-alkenyloxy, fluoro-C₂-C₄-alkoxy, C₄-C₈-cycloalkyloxy, C₃-C₈-cycloalkyl-C₁-C₄-alkoxy, halogen, aryl-C₁-C₄-alkylthio, heteroaryl-C₁-C₄-allcylthio, aryl-C₁-C₄-alkylamino, heteroaryl-C₁-C₄-alkylamino, heteroaryl and aryl;
and any aryl and heteroaryl residue, alone or as part of another group, in R₂ may be substituted in one or more positions, preferably one or two, independently of each other by C₁₋₄-alkyl, C₁₋₄-alkoxy, C₁₋₄-alkylthio, C₂₋₄-acyl, C₁₋₄-alkylsulphonyl, cyano, nitro, hydroxy, C₂₋₆-alkenyl, C₂₋₆-alkynyl, fluoromethyl, trifluoromethyl, trifluoromethoxy, halogen, -N(R₅)(R₆), aryl, aryloxy, arylthio, aryl-C₁₋₄-alkyl, aryl-C₂₋₄-alkenyl, aryl-C₂₋₄-alkynyl, heteroaryl, heteroaryloxy, heteroarylthio or heteroaryl-C₁₋₄-alkyl, aryl-C₁₋₄-alkoxy, aryloxy-C₁₋₄-alkyl, dimethylamino-C₂-₄-alkoxy; and
any aryl or heteroaryl residue as substituents on aryl or heteroaryl, alone or as part of another group, in R₂ in turn may be substituted in one or more postions, preferably one, independently of each other by C₁₋₄-alkyl, C₁₋₄-alkoxy, halogen, trifluoromethyl, cyano, hydroxy or dimethylamino;
with the proviso that:
(i) R₂ is other than phenylthio, phenylmethylthio, phenyl or phenyl substituted by halogen;
(ii) R₂ is halogen only when the piperazine ring is 2-methylpiperazin-1-yl or 2-ethyl-piperazin-1-yl;
R₃ is H or C₁₋₄-alkyl, allyl, 2-hydroxyethyl or 2-cyanoethyl;
R₅ and R₆ independently of each other are hydrogen, methyl or ethyl, or together with the nitrogen atom to which they are bound form a pyrrolidine, piperazine, morpholine, thiomorpholine or a piperidine ring;
R₇ is hydrogen or C₁₋₄-alkyl;
and pharmaceutically acceptable salts, hydrates, geometrical isomers, tautomers and optical isomers thereof.

2. The compound according to claim 1 wherein R₃ is hydrogen.

3. The compound according to claim 1 wherein R₇ is selected from hydrogen, methyl or ethyl.

4. The compound according to claim 1 wherein R₇ is methyl and where said methyl is attached to the C2-position of the piperazine ring.

5. The compound according to claim 1 wherein R₇ is hydrogen.

6. The compound according to claim 1, which is selected from the group consisting of:
2-(Benzyloxy)-6-(1-piperazinyl)pyrazine,
2-[(2-Methoxybenzyl)oxy]-6-(1-piperazinyl)pyrazine,
2-[(3-Methoxybenzyl)oxy]-6-(1-piperazinyl)pyrazine,
2-[(3,5-Difluorobenzyl)oxy]-6-(1-piperazinyl)pyrazine,
2-(1-Naphthylmethoxy)-6-(1-piperazinyl)pyrazine,
2-(1-Phenylethoxy)-6-(1-piperazinyl)pyrazine,
2-[1-(3-Fluorophenyl)ethoxy]-6-(1-piperazinyl)pyrazine,
2-[1-(2-Methoxyphenyl)ethoxy]-6-(1-piperazinyl)pyrazine,
2-(3,4-Dihydro-2*H*-chromen-4-yloxy)-6-(1-piperazinyl)pyrazine,
2-(2-Phenylethoxy)-6-(1-piperazinyl)pyrazine,
2-[(2-Phenoxybenzyl)oxy]-6-(1-piperazinyl)pyrazine,
2-[2-(3-Chlorophenyl)ethoxy]-6-(1-piperazinyl)pyrazine,
2-[2-(2-Methoxyphenyl)ethoxy]-6-(1-piperazinyl)pyrazine,
2-[2-(3-Methoxyphenyl)ethoxy]-6-(1-piperazinyl)pyrazine,
2-[2-(4-Methoxyphenyl)ethoxy]-6-(1-piperazinyl)pyrazine,
2-[2-(2,5-Dimethoxyphenyl)ethoxy]-6-(1-piperazinyl)pyrazine,
2-[(2-Phenylethyl)sulfanyl]-6-(1-piperazinyl)pyrazine,
2-[(5-Fluoro-2-methoxybenzyl)oxy]-6-(1-piperazinyl)pyrazine,
2-[(3-Cyanobenzyl)oxy]-6-(1-piperazinyl)pyrazine,
2-[(2-Chlorobenzyl)sulfanyl]-6-(1-piperazinyl)pyrazine,
2-[2-(4-Dimethylaminophenyl)ethoxy]-6-(1-piperazinyl)pyrazine,
2-[2-(1*H*-Indol-3-yl)ethoxy]-6-(1-piperazinyl)pyrazine,
2-[2-(1*H*-Indol-1-yl)ethoxy]-6-(1-piperazinyl)pyrazine,
2-Benzyl-6-(1-piperazinyl)pyrazine,
2-[(3,5-Dimethoxybenzyl)oxy]-6-(1-piperazinyl)pyrazine,
1-[6-(Benzyloxy)-2-pyrazinyl]-2-methylpiperazine,
1-[6-(Benzyloxy)-2-pyrazinyl]-2-ethylpiperazine
1-[6-(Benzyloxy)-2-pyrazinyl]-trans-2,5-dimethylpiperazine.
2-[2-(2-Fluorophenyl)ethoxy]-6-(1-piperazinyl)pyrazine,
2-(2,3-Dihydro-1*H*-inden-1-ylmethoxy)-6-(1-piperazinyl)pyrazine
2-(4-Phenoxybutoxy)-6-(1-piperazinyl)pyrazine.
2-[(5-Phenoxypentyl)oxy]-6-(1-piperazinyl)pyrazine.
2-[(2,5-Dimethoxybenzyl)oxy]-6-(1-piperazinyl)pyrazine.
2-{[2-(2-Phenylethyl)benzyl]oxy}-6-(1-piperazinyl)pyrazine
(2*R*)-1-[6-(Benzyloxy)-2-pyrazinyl]-2-methylpiperazine,
2-[2-(2,6-Difluorophenoxy)ethoxy]-6-(1-piperazinyl)pyrazine
2-[2-(2-Naphthyloxy)ethoxy]-6-(1-piperazinyl)pyrazine
2-(1-Methyl-2-phenylethoxy)-6-(1-piperazinyl)pyrazine
2-{[2-(Phenoxymethyl)benzyl]oxy}-6-(1-piperazinyl)pyrazine
2-[(5-Fluoro-2-methoxybenzyl)oxy]-6-(1-piperazinyl)pyrazine
2-[(2,5-Difluorobenzyl)oxy]-6-(1-piperazinyl)pyrazine
2-[(2-Fluorobenzyl)oxy]-6-(1-piperazinyl)pyrazine
2-(Benzo[*b*]thiophen-3-ylmethoxy)-6-(1-piperazinyl)pyrazine,
2-[2-(5-Methyl-2-phenyl-oxazol-4-yl)-ethoxy]-6-(1-piperazinyl)pyrazine,
2-[1-(2,6-Difluoro-phenyl)-ethoxy]-6-(1-piperazinyl)pyrazine,
2-(2-Naphthalen-2-yl-ethoxy)-6-(1-piperazinyl)pyrazine,
2-[3-(Naphthalen-2-yloxy)-propoxy]-6-(1-piperazinyl)pyrazine,
2-[2-(7-Methoxy-naphthalen-2-yloxy)-ethoxy]-6-(1-piperazinyl)pyrazine,
2-[5-(4-Chlorophenyl)-2-methylfuran-3-ylmethoxy]-6-(1-piperazinyl)pyrazine,
2-(1*H*-Indol-4-ylmethoxy)-6-(1-piperazinyl)pyrazine,
and their pharmacologically acceptable salts and solvates.

7. A pharmaceutical composition comprising a compound according to any one of claims 1 to 6 as an active ingredient, together with a pharmaceutically acceptable carrier.

8. Use of a compound according to any one of claims 1 to 6 in the manufacture of a medicament for the prophylaxis or treatment of a serotonin-related medical condition.

9. The use according to claim 8, wherein the medical condition is related to the 5-HT_{2c} receptor.

10. The use according to claim 8 wherein the medical condition is an eating disorder.

11. The use according to claim 8, wherein the medical condition is obesity.

12. The use according to claim 8, wherein the medical condition a memory disorder.

13. The use according to claim 8, wherein the medical condition is a mood disorder.

14. The use according to claim 8, wherein the medical condition is an anxiety disorder.

15. The use according to claim 8, wherein the medical condition is selected from sexual dysfunctions, epilepsy and urinary disorders.

16. The use according to claim 8, wherein the medical condition is pain.

17. The use according to claim 8, wherein the medical condition is substance abuse.

18. The use according to claim 8, wherein the medical condition is schizophrenia.

## Patentansprüche

1. Verbindung der allgemeinen Formel (II): 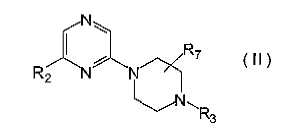 worin
R₂ ausgewählt ist aus der Gruppe bestehend aus Aryl-C₁₋₆-alkyl, Aryl-C₁₋₆-alkoxy, Heteroaryl-C₁₋₆-alkoxy, Aryloxy-C₂₋₆-alkoxy, Heteroaryloxy-C₂₋₆-alkoxy, 1-Indanyloxy, 2-Indanyloxy, Aryloxy, Heteroaryloxy, Arylthio, Heteroarylthio, C₅₋₆-Cycloalkylthio, C₅₋₈-Alkoxy_{,} C₅₋₈-Alkylthio, C₃₋₆-Alkinyloxy, C₃₋₆-Alkenyloxy, Fluor-C₂₋₄-alkoxy, C₄₋₈-Cycloalkyloxy, C₃₋₈-Cycloalkyl-C₁₋₄-alkoxy, Halogen, Aryl-C₁₋₄-alkylthio, Heteroaryl-C₁₋₄-alkylthio, Aryl-C₁₋₄-alkylamino, Hetetoaryl-C₁₋₄-alkylamino, Heteroaryl und Aryl;
und jeder Aryl- und Heteroarylrest, alleine oder als Teil einer anderen Gruppe, in R² an einer oder mehreren Positionen, vorzugsweise einer oder zwei, jeweils unabhängig voneinander mit C₁₋₄-Alkyl, C₁₋₄-Alkoxy, C₁₋₄-Alkylthio, C₂₋₄-Acyl, C₁₋₄-Alkylsulfonyl, Cyano, Nitro, Hydroxy, C₂₋₆-Alkenyl, C₂₋₆-Alkinyl, Fluormethyl, Trifluormethyl, Trifluormethoxy, Halogen, -N(R₅) (R₆), Aryl, Aryloxy, Arylthio, Aryl-C₁₋₄-alkyl, Aryl-C₂₋₄-alkenyl, Aryl-C₂₋₄-alkinyl, Heteroaryl, Heteroaryloxy, Heteroarylthio, Heteroaryl-C₁₋₄-alkyl, Aryl-C₁₋₄-alkoxy, Aryloxy-C₁₋₄-alkyl oder Dimethylamino-C₂₋₄-alkoxy substituiert sein kann; und
jeder Aryl- oder Heteroarylrest als Substituent an Aryl oder Heteroaryl, alleine oder als Teil einer anderen Gruppe, in R² andererseits an einer oder mehreren Positionen, vorzugsweise einer, jeweils unabhängig voneinander mit C₁₋₄-Alkyl, C₁₋₄-Alkoxy, Halogen, Trifluormethyl, Cyano, Hydroxy oder Dimethylamino substituiert sein kann;
unter der Voraussetzung, dass
(i) R₂ kein Phenylthio, Phenylmethylthio, Phenyl oder mit Halogen substituiertes Phenyl ist;
(ii) R₂ nur dann Halogen ist, wenn der Piperazinring 2-Methylpiperazin-1-yl oder 2-Ethylpiperazin-1-yl ist;
R₃ H oder C₁₋₄-Alkyl, Allyl, 2-hydroxymethyl oder 2-Cyanoethyl ist;
R₅ und R₆ jeweils unabhängig voneinander Wasserstoff, Methyl oder Ethyl sind oder zusammen mit dem Stickstoffatom, an das sie gebunden sind, einen Pyrrolidin-, Piperazin- Morpholin-, Thiomorpholin- oder Piperidinring bilden;
R₇ Wasserstoff oder C₁₋₄-Alkyl ist;
oder pharmazeutisch annehmbare Salze, Hydrate, geometrische Isomere, Tautomere und optische Isomere davon.

2. Verbindung gemäss Anspruch 1, worin R₃ Wasserstoff ist.

3. Verbindung gemäss Anspruch 1, worin R₇ aus Wasserstoff, Methyl oder Ethyl ausgewählt ist.

4. Verbindung gemäss Anspruch 1, worin R₇ Methyl ist und wobei das Methyl an der C2-Position des Piperazinrings angefügt ist.

5. Verbindung gemäss Anspruch 1, worin R₇ Wasserstoff ist.

6. Verbindung gemäss Anspruch 1, ausgewählt aus der Gruppe bestehend aus:
2-(Benzyloxy)-6-(1-piperazinyl)pyrazin,
2-[(2-Methoxybenzyl)oxy]-6-(1-piperazinyl)pyrazin,
2-[(3-Methoxybenzyl)oxy]-6-(1-piperazinyl)pyrazin,
2-[(3,5-Difluorbenzyl)oxy]-6-(l-piperazinyl)pyrazin,
2-(1-Naphthylmethoxy)-6-(1-piperazinyl)pyrazin,
2-(1-Phenylethoxy)-6-(1-piperazinyl)pyrazin,
2-[1-(3-Fluorphenyl)ethoxy]-6-(1-piperazinyl)pyrazin,
2-[1-(2-Methoxyphenyl)ethoxy]-6-(1-piperazinyl) - pyrazin,
2-(3,4-Dihydro-2H-chromen-4-yloxy)-6-(1-piperazinyl)-pyrazin,
2-(2-Phenylethoxy)-6-(1-piperazinyl)pyrazin,
2-[(2-Phenoxybenzyl)oxy]-5-(7-piperazinyl)pyrazin, 2-[2-(3-Chlorphenyl)ethoxy]-6-(1-piperazinyl)pyrazin,
2-[2-(2-Methoxyphenyl)ethoxy]-6-(1-piperazinyl)-pyrazin,
2-[2-(3-Methoxyphenyl)ethoxy]-6-(1-piperazinyl)-pyrazin,
2-[2-(4-Methoxyphenyl)ethoxy]-6-(1-piperazinyl)-pyrazin,
2-[2-(2,5-Dimethoxyphenyl)ethoxy]-6-(1-piperazinyl)-pyrazin,
2-[(2-Phenylethyl)sulfanyl]-6-(1-piperazinyl)pyrazin,
2-[(5-Fluor-2-methoxybenzyl)oxy]-6-(1-piperazinyl)-pyrazin,
2- (3-Cyanobenzyl)oxy] -6-(1-piperazinyl)pyrazin,
2- [(2-Chlorbenzyl) sulfanyl]-6-(1-piperazinyl)pyrazin,
2-[2-(4-Dimethylaminophenyl)ethoxy]-6-(1-piperazinyl)pyrazin,
2-[2-(1H-Indol-3-yl)ethoxy]-6-(1-piperazinyl)pyrazin,
2-[2-(1H-Indol-1-yl)ethoxy]-6-(1-piperazinyl)pyrazin,
2-Benzyl-6-(1-piperazinyl)pyrazin,
2-[(3,5-Dimethoxybenzyl.)oxy]-6-(1-piperazinyl)-pyrazin,
1-[6-(Benzyloxy)-2-pyrazinyl]-2-methylpiperazin,
1-[6-(Benzyloxy)-2-pyrazinyl]-2-ethylpiperazin,
1-[6-(Benzyloxy)-2-pyrazinyl]-trans-2,5-dimethylpiperazin,
2-[2-(2-Fluorphenyl)ethoxy]-6-(1-piperazinyl)pyrazin,
2-(2,3-Dihydro-1H-inden-1-ylmethoxy)-6-(1-piperazinyl)pyrazin,
2-(A-Phenoxybutoxy)-6-(1-piperazinyl)pyrazin,
2-[(5-Phenoxypentyl)oxy]-6-(1-pipexazinyl)pyrazin, 2-[(2,5-Dimethoxybenzyl)oxy]-6-(1-piperazinyl)-pyrazin,
2-{[2-(2-Phenylethyl)benzyl]oxy}-6-(1-piperazinyl)-pyrazin,
(2R)-1- [6-(Benzyloxy)-2-pyrazinyl]-2-methylpiperazin,
2-[2-(2,6-Difluorphenoxy)ethoxy]-6-(1-piperazinyl)-pyrazin,
2-[2-(2-Naphthyloxy)ethoxy]-6-(1-piperazinyl)pyrazin,
2-(1-Methyl-2-phenylethoxy)-6-(1-piperazinyl)pyrazin,
2-{[12-(Phenoxymethyl)benzyl]oxy}-6-(1-piperazinyl)-pyrazin,
2-[(5-Fluor-2-methoxybenzyl)oxy]-6-(1-piperazinyl)-pyrazin,
2-[(2,5-Difluorbenzyl)oxy]-6-(1-piperazinyl)pyrazin, 2-[(2-Fluorbenzyl)oxy]-6-(1-piperazinyl)pyrazin,
2-(Benzo[b]thiophen-3-ylmethoxy)-6-(1-piperazinyl)" pyrazin,
2-[2-(5-Methyl-2-phenyloxazol-4-yl)ethoxy]-6-(1-piperazinyl)pyrazin,
2-[1-(2,6-Difluorphenyl)ethoxy]-6-(1-piperazinyl)-pyrazin,
2-(2-Naphthalin-2-ylethoxy)-6-(1-piperazinyl)pyrazin,
2-[3-(Naphthalin-2-yloxy)propoxy]-6-(1-piperazinyl)-pyrazin,
2[2-(7-Methoxynaphthalin-2-yloxy)ethoxy]-6-(1-piperazinyl)pyrazin,
2-[5-(4-Chlorphenyl)-2-methylfuran-3-ylmethoxy]-6-(1-piperazinyl)pyrazin,
2-(1H-Indol-4-ylmethoxy)-6-(1-piperazinyl)pyrazin,
und ihre pharmakologisch annehmbaren Salze und Solvate.

7. Pharmazeutische Zusammensetzung, umfassend eine Verbindung gemäss irgendeinem der Ansprüche 1 bis 6 als aktiven Bestandteil zusammen mit einem pharmazeutisch annehmbaren Träger.

8. Verwendung einer Verbindung gemäss irgendeinem der Anspruche 1 bis 6 zur Herstellung eines Medikaments zur Prophylaxe oder Behandlung von Serotoninabhängigen medizinischen Zuständen.

9. Verwendung gemäss Anspruch 8, wobei der medizinische Zustand von dem 5-HT_{2c}-Rezeptor abhängt.

10. Verwendung gemäss Anspruch 8, wobei der medizinische Zustand eine Essstörung ist.

11. Verwendung gemäss Anspruch 8, wobei der medizinische Zustand Fettleibigkeit ist.

12. Verwendung gemäß Anspruch 8, wobei der medizinische Zustand eine Gedächtnisstörung ist.

13. Verwendung gemäss Anspruch 8, wobei der medizinische Zustand eine Gemütszustandsstörung ist.

14. Verwendung gemäss Anspruch 8, wobei der medizinische Zustand eine Angststörung ist.

15. Verwendung gemäss Anspruch 8, wobei der medizinische Zustand aus sexuellen Funktionsstörungen, Epilepsie und Harnstörungen ausgewählt ist.

16. Verwendung gemäss Anspruch 8, wobei der medizinische Zustand Schmerz ist.

17. Verwendung gemäss Anspruch 8, wobei der medizinische Zustand Drogenmissbrauch ist.

18. Verwendung gemäss Anspruch 8, wobei der medizinische Zustand Schizophrenie ist.

## Revendications

1. Composé de formule générale (II): 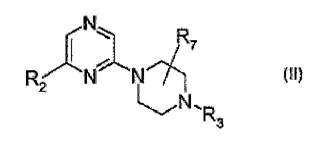 dans laquelle
R₂ est choisi dans le groupe constitué par les radicaux aryl-alkyle en C₁-C₆, aryl-alcoxy en C₁-C₆, hétéroaryl-alcoxy en C₁-C₆, aryloxy-alcoxy en C₂-C₆, hétéroaryloxy-alcoxy en C₂-C₆, 1-indanyloxy, 2-indanyloxy, aryloxy, hétéroaryloxy, arylthio, hétéroarylthio, cycloalkylthio en C₅-C₆, alcoxy en C₅-C₆, alkylthio en C₅-C₈, alcynyloxy en C₃-C₆, alcényloxy en C₃-C₆, fluoro-alcoxy en C₂-C₄, cycloalkyloxy en C₄-C₈, cycloalkyl(en C₃-C₈)-alcoxy en C₁-C₄, halogène, aryl-alkyl(en C₁-C₄)thio, hétéroaryl-alkyl(en C₁-C₄)thio, aryl-alkyl(en C₁-C₄)amino, hétéroaryl-alkyl(en C₁-C₄)amino, hétéroaryle et aryle;
et tout résidu aryle ou hétéroaryle dans R₂, seul ou en tant que partie d'un autre groupe, peut être substitué en une ou plusieurs positions, de préférence, une ou deux positions, indépendamment les unes des autres, par un radical alkyle en C₁-C₄, alcoxy en C₁-C₄, alkylthio en C₁-C₄, acyle en C₂-C₄, alkyl(en C₁-C₄)sulfonyle, cyano, nitro, hydroxy, alcényle en C₂-C₆, alcynyle en C₂-C₆, fluorométhyle, trifluorométhyle, trifluorométhoxy, halogène, -N(R₅)(R₆), aryle, aryloxy, arylthio, aryl-alkyle en C₁-C₄, aryl-alcényle en C₂-C₄, aryl-alcynyle en C₂-C₄, hétéroaryle, hétéroaryloxy, hétéroarylthio ou hétéroaryl-alkyle en C₁-C₄, aryl-alcoxy en C₁-C₄, aryloxy-alkyle en C₁-C₄, diméthylamino-alcoxy en C₂-C₄; et
tout résidu aryle ou hétéroaryle dans R₂, servant de substituants sur le groupe aryle ou hétéroaryle, seul ou en tant que partie d'un autre groupe, peut être quant à lui substitué en une ou plusieurs positions, de préférence, une position, indépendamment les unes des autres, par une radical alkyle en C₁-C₄, alcoxy en C₁-C₄, halogène, trifluorométhyle, cyano, hydroxy ou diméthylamino;
à condition que:
(i) R₂ soit différent d'un groupe phénylthio, phénylméthylthio, phényle ou phényle substitué par un halogène;
(ii) R₂ soit un halogène uniquement lorsque le noyau pipérazine est le 2-methylpiperazin-1-yle ou le 2-éthyl-pipérazin-1-yle;
R₃ représente H ou un groupe alkyle en C₁-C₄, allyle, 2-hydroxyéthyle ou 2-cyanoéthyle;
R₅ et R₆ représentent, indépendamment l'un de l'autre, un atome d'hydrogène, un groupe méthyle ou éthyle, ou conjointement avec l'atome d'azote auquel ils sont liés, forment un noyau pyrrolidine, pipérazine, morpholine, thiomorpholine ou pipéridine;
R₇ est un atome d'hydrogène ou un groupe alkyle en C₁-C₄,
et les sels, hydrates, isomères géométriques, tautomères et isomères optiques pharmaceutiquement acceptables de celui-ci.

2. Composé selon la revendication 1, dans lequel R₃ est un atome d'hydrogène.

3. Composé selon la revendication 1, dans lequel R₇ est choisi parmi l'atome d'hydrogène, le groupe méthyle ou éthyle.

4. Composé selon la revendication 1, dans lequel R₇ est un méthyle et où ledit méthyle est lié en position C2 du noyau pipérazine.

5. Composé selon la revendication 1, dans lequel R₇ est un atome d'hydrogène.

6. Composé selon la revendication 1, qui est choisi dans le groupe constitué par:
la 2-(benzyloxy)-6-(1-pipérazinyl)pyrazine,
la 2-[(2-méthoxybenzyl)oxy]-6-(1-pipérazinyl)pyrazine,
la 2-[(3-méthoxybenzyl)oxy]-6-(1-pipérazinyl)pyrazine,
la 2-[(3,5-difluorobenzyl)oxy]-6-(1-pipérazinyl)pyrazine,
la 2-(1-naphtylméthoxy)-6-(1-pipérazinyl)pyrazine,
la 2-(1-phényléthoxy)-6-(1-pipérazinyl)pyrazine,
la 2-[1-(3-fluorophényl)éthoxy]-6-(1-pipérazinyl)pyrazine,
la 2-[1-(2-méthoxyphényl)éthoxy]-6-(1-piperazinyl)pyrazine,
la 2-(3,4-dihydro-2*H*-chromén-4-yloxy)-6-(1-pipérazinyl)pyrazine,
la 2-(2-phényléthoxy)-6-(1-pipérazinyl)pyrazine,
la 2-[(2-phénoxybenzyl)oxy]-6-(1-pipérazinyl)pyzazine,
la 2-[2-(3-chlorophényl)éthoxy]-6-(1-pipérazinyl)pyrazine,
la 2-[2-(2-méthoxyphényl)éthoxy]-6-(1-pipérazinyl)pyrazine,
la 2-[2-(3-méthoxyphényl)éthoxy]-6-(1-pipérazinyl)pyrazine,
la 2-[2-(4-méthoxyphényl)éthoxy]-6-(1-pipérazinyl)pyrazine,
la 2-[2-(2,5-diméthoxyphényl)éthoxy]-6-(1-pipérazinyl)pyrazine,
la 2-[(2-phényléthyl)sulfanyl]-6-(1-pipérazinyl)pyrazine,
la 2-[(5-fluoro-2-méthoxybenzyl)oxy]-6-(1-pipérazinyl)pyrazine,
la 2-[(3-cyanobenzyl)oxyl-6-(1-pipérazinyl)pyrazine,
la 2-[(2-chlorobenyl)sulfanyl]-6-(1-pipérazinyl)pyrazine,
la 2-[2-(4-diméthylaminophényl)éthoxy]-6-(1-pipérazinyl)pyrazine,
la 2-[2-(1*H*-indol-3-yl)éthoxy]-6-(1-pipérazinyl)pyrazine,
la 2-[2-(1*H*-indol-1-yl)éthoxy]-6-(1-pipérazinyl)pyrazine,
la 2-benzyl-6-(1-pipérazinyl)pyrazine,
la 2-[(3,5-diméthoxybenzyl)oxy]-6-(1-pipérazinyl)pyrazine,
la 1-[6-benzyloxy)-2-pyrazinyl]-2-méthylpipérazine,
la 1-[6-benzyloxy)-2-pyrazinyl]-2-éthylpipérazine,
la 1-[6-benzyloxy)-2-pyrazinyl]-trans-2,5-diméthylpipérazine,
la 2-[2-(2-fluorophényl)éthoxy]-6-(1-pipérazinyl)pyrazine,
la 2-(2,3-dibydro-1*H*-indén-1-ylméthoxy)-6-(1-piperazinyl)pyrazine,
la 2-(4-phénoxybutoxy)-6-(1-pipérazinyl)pyrazine,
la 2-[(5-phénoxypentyl)oxy]-6-(1-pipérazinyl)pyrazine,
la 2-[(2,5-diméthoxybenzyl)oxy]-6-(1-pipérazinyl)pyrazine,
la 2-{[2-(2-phényléthyl)benzyl]oxy}-6-(1-pipérazinyl)pyrazine,
la (2*R*)-1-[6-(benzyloxy)-2-pyrazinyl]-2-méthylpipérazine,
la 2-[2-(2,6-difluorophénoxy)éthoxy]-6-(1-pipérazinyl)pyrazine,
la 2-[2-(2-naphtyloxy)éthoxy]-6-(1-pipérazinyl)pyrazine,
la 2-(1-méthyl-2-phényléthoxy)-6-(1-pipérazinyl)pyrazine,
la 2-{[2-(phénoxyméthyl)benzyl]oxy}-6-(1-pipérazinyl)pyrazine,
la 2-[(5-fluoro-2-méthoxybenzyl)oxy]-5-(1-pipérazinyl)pyrazine,
la 2-[(2,5-difluorobenzyl)oxy]-6-(1-pipérazinyl)pyrazine,
la 2-[(2-fluorobenzyl)oxy]-6-(1-pipérazinyl)pyrazine,
la 2-(benzo[*b*]thiophén-3-ylméthoxy)-6-(1-pipérazinyl)pyrazine,
la 2-[2-(5-methyl-2-phényl-oxazol-4-yl)éthoxy]-6-(1-pipérazinyl)pyrazine,
la 2-[1-(2,6-difluoro-phényl)éthoxy]-6-(1-pipérazinyl)pyrazine,
la 2-(2-naphtalén-2-yl-éthoxy)-6-(1-pipérazinyl)pyrazine,
la 2-[3-(naphtalén-2-yloxy)propoxy]-6-(1-pipérazinyl)pyrazine,
la 2-[2-(7-méthoxy-naphtalén-2-yloxy)éthoxy]-6-(1-pipérazinyl)pyrazine,
la 2-[5-(4-chlorophényl)-2-methylfuran-3-ylméthoxy]-6-(2-pipérazinyl)-pyrazine,
la 2-(1*H*-indol-4-ylméthoxy]-6-(1-pipérazinyl)pyrazine,
et leurs sels et solvates acceptables sur le plan pharmacologique.

7. Composition pharmaceutique comprenant un composé selon l'une quelconque des revendications 1 à 6 en tant que principe actif, conjointement avec un véhicule pharmaceutiquement acceptable.

8. Utilisation d'un composé selon l'une quelconque des revendications 1 à 6 dans la fabrication d'un médicament destiné à la prophylaxie et au traitement d'un état pathologique associé à la sérotonine.

9. Utilisation selon la revendication 8, où l'état pathologique est associé au récepteur 5-HT_{2c}.

10. Utilisation selon la revendication 8, où l'état pathologique est un trouble de l'alimentation.

11. Utilisation selon la revendication 8, où l'état pathologique est l'obésité.

12. Utilisation selon la revendication 8, où l'état pathologique est un trouble de la mémoire.

13. Utilisation selon la revendication 8, où l'état pathologique est un trouble de l'humeur.

14. Utilisation selon la revendication 8, où l'état pathologique est un trouble de l'anxiété.

15. Utilisation selon la revendication 8, où l'état pathologique est choisi parmi les troubles de la sexualité, l'épilepsie et les troubles urinaires.

16. Utilisation selon la revendication 8, où l'état pathologique est la douleur.

17. Utilisation selon la revendication 8, où l'état pathologique est la pharmacodépendance.

18. Utilisation selon la revendication 8, ou l'état pathologique est la schizophrénie.
